# EUROPEAN PATENT SPECIFICATION

(11) **EP 1 866 647 B1**
(45) Date of publication and mention of the grant of the patent: **22.04.2015**
(21) Application number: 06721313.2
(22) Date of filing: 31.03.2006
(51) Int. Cl.: C07K 16/40, G01N 33/50, G01N 33/573, G01N 33/574

(54) **A METHOD OF DIAGNOSIS AND TREATMENT AND AGENTS USEFUL FOR SAME**
DIAGNOSE- UND BEHANDLUNGSVERFAHREN SOWIE DAFÜR GEEIGNETE MITTEL
PROCÉDÉ POUR LE DIAGNOSTIC ET LE TRAITEMENT ET AGENTS UTILES POUR CELUI-CI

(30) Priority: 01.04.2005 AU 2005901613
(43) Date of publication of application: 19.12.2007
(73) Proprietor: MEDVET SCIENCE PTY. LTD., Stepney, South Australia 5069 (AU)
(72) Inventor: BROWN, Michael, Paul, St. Georges, South Australia 5064 (AU)
(74) Representative: Gardner, Rebecca Katherine
(86) International application number: PCT/AU2006/000431
(87) International publication number: WO 2006/102716

(56) References cited:
- US-B1- 6 639 057
- KELLAND ET AL: "Telomerase: biology and phase I trials" LANCET ONCOLOGY, LANCET PUBLISHING GROUP, LONDON, GB, vol. 2, no. 2, 1 February 2001 (2001-02-01), pages 95-102, XP005063058 ISSN: 1470-2045
- EISO HIYAMA ET AL: "Telomerase detection in the diagnosis and prognosis of cancer" CYTOTECHNOLOGY, KLUWER ACADEMIC PUBLISHERS, DO, vol. 45, no. 1-2, 1 June 2004 (2004-06-01), pages 61-74, XP019236837 ISSN: 1573-0778
- SAPI EVA ET AL: "Detection of telomerase-positive circulating epithelial cells in ovarian cancer patients." CANCER DETECTION AND PREVENTION, vol. 26, no. 2, 2002, pages 158-167, XP002497894 ISSN: 0361-090X
- WAGURI NOBUO ET AL: "Sensitive and specific detection of circulating cancer cells in patients with hepatocellular carcinoma; detection of human telomerase reverse transcriptase messenger RNA after immunomagnetic separation." CLINICAL CANCER RESEARCH : AN OFFICIAL JOURNAL OF THE AMERICAN ASSOCIATION FOR CANCER RESEARCH 1 AUG 2003, vol. 9, no. 8, 1 August 2003 (2003-08-01), pages 3004-3011, XP002497895 ISSN: 1078-0432
- YAN P. ET AL.: 'Immunohistochemical localization of hTERT protein in human tissues' HISTOCHEMISTRY AND CELL BIOLOGY vol. 121, no. 5, May 2004, pages 391 - 397, XP003018774
- KUSHNER D.M. ET AL.: '2-5A antisense directed against telomerase RNA produces apoptosis in ovarian cancer cells' GYNACOLOGIC ONCOLOGY vol. 76, 2000, pages 183 - 192, XP000972332
- COLANGELO D. ET AL.: 'Cis-[Pt(Cl)2(pyridine)(5-SO3isoquinoline)] complex, a selective inhibitor of telomerase enzyme' BIOMETALS vol. 16, 2003, pages 553 - 560, XP008121771
- ZEZHANG T. ET AL.: 'Targeted therapy of human laryngeal squamous cell carcinoma in vitro by antisense oligonucleotides directed against telomerase reverse transcriptase mRNA' THE JOURNAL OF LARYNGOLOGY AND OTOLOGY vol. 119, 2005, pages 92 - 96, XP008121824
- NAKAJIMA A. ET AL.: 'Telomerase inhibition enhances apoptosis in human acute leukemia cells: possibility of antitelomerase therapy' LEUKEMIA vol. 17, 2003, pages 560 - 567, XP008121772
- KOMATA T. ET AL.: 'Telomerase as a therapeutic target for malignant gliomas' ONCOGENE vol. 21, 2002, pages 656 - 663, XP008121773
- LUDWIG A. ET AL.: 'Ribozyme cleavage of telomerase mRNA sensitizes breast epithelial cells to inhibitors of topisomerase' CANCER RESEARCH vol. 61, 2001, pages 3053 - 3061, XP008121774
- ZHANG YUAN ET AL.: 'Inhibition of telomerase activity with hTERT antisense increases the effect of CDDP-induced apoptosis in myeloid leukemia' THE HEMATOLOGY JOURNAL vol. 3, 2002, pages 201 - 205, XP008121829
- MO Y. ET AL.: 'Simultaneous targeting of telomeres and telomerase as a cancer therapeutic approach' CANCER RESEARCH vol. 63, 2003, pages 579 - 585, XP008121775
- PITTS A.E. ET AL.: 'Inhibition of human telomerase by 2'-O-methyl-RNA' PROCEEDINGS OF THE NATIONAL ACADEMY OF SCIENCES (USA) vol. 95, 2001, pages 11549 - 11554, XP002242128
- ARAI J. ET AL.: 'Identification of human telomerase reverse transcriptase-derived peptides that induce HLA-A24-restricted antileukemia cytotoxic T lymphocytes' BLOOD vol. 97, no. 9, 2001, pages 2903 - 2907, XP002980964
- VONDERHEIDE R.H. ET AL.: 'The telomerase catalytic subunit is a widely expressed tumor-associated antigen recognized by cytotoxic T lymphocytes' IMMUNITY vol. 10, 1999, pages 673 - 679, XP002950257
- MINEV B. ET AL.: 'Cytotoxic T cell immunity against telomerase reverse transcriptase in humans' PROCEEDINGS OF THE NATIONAL ACADEMY OF SCIENCES (USA) vol. 7, 2000, pages 4796 - 4801, XP002155017

### SEQUENCE LISTING

<110> Medvet Science Pty. Ltd.
<120> A method of diagnosis and treatment and agents useful for same
<130> 12729120/TDO
<150> 2005901613
   <151> 2005-01-04
<160> 5
<170> PatentIn version 3.1
<210> 1
   <211> 26
   <212> DNA
   <213> primer
<400> 1
   gaagggcgta ggcgccgtgc ttttgc 26
<210> 2
   <211> 27
   <212> DNA
   <213> primer
<400> 2
   gtttgctcta gaatgaacgg tggaagg 27
<210> 3
   <211> 28
   <212> DNA
   <213> primer
<400> 3
   gcctgggagg ggtggtggct attttttg 28
<210> 4
   <211> 24
   <212> DNA
   <213> primer
<400> 4
   cggagtcaac ggatttggtc gtat 24
<210> 5
   <211> 24
   <212> DNA
   <213> primer
<400> 5
   agccttctcc atggtggtga agac 24

## Description

### FIELD OF THE INVENTION

The present invention relates generally to a method of screening for the level of neoplastic cell death in a subject. More particularly, the present invention provides a method of screening for the level of neoplastic cell death by detecting the level of expression of telomerase RNA, mRNA or protein in dead cells which have lost membrane integrity in said subject or in a biological sample derived from said subject. The method of the present invention is useful in a range of applications including, but not limited to, assessing a neoplastic condition, monitoring the progression of such a condition, assessing the effectiveness of a therapeutic agent or therapeutic regime and predicting the likelihood of a subject either progressing to a more advanced disease state or entering a remissive state. The present invention also relates to diagnostic agents useful for detecting telomerase protein and/or nucleic acid molecules.

The present application also discloses a means for therapeutic targetting, either *in vitro* or *in vivo,* of drugs such as inhibitors of cellular proliferation. The ability to target dead or dying neoplastic cells is useful in a range of immunotherapeutic and immunoprophylactic treatments due to the fact that said dead or dying neoplastic cells localise with live neoplastic cells.

### BACKGROUND OF THE INVENTION

Bibliographic details of the publications referred to by author in this specification are collected alphabetically at the end of the description.

The reference to any prior art in this specification is not, and should not be taken as, an acknowledgment or any form of suggestion that that prior art forms part of the common general knowledge in Australia.

Malignant tumours, or cancers, grow in an uncontrolled manner, invade normal tissues, and often metastasize and grow at sites distant from the tissue of origin. In general, cancers are derived from one or only a few normal cells that have undergone a poorly understood process called malignant transformation. Cancers can arise from almost any tissue in the body. Those derived from epithelial cells, called carcinomas, are the most common kinds of cancers. Sarcomas are malignant tumours of mesenchymal tissues, arising from cells such as fibroblasts, muscle cells, and fat cells. Solid malignant tumours of lymphoid tissues are called lymphomas, and marrow and blood-borne malignant tumours of lymphocytes and other hematopoietic cells are called leukemias.

Cancer is one of the three leading causes of death in industrialised nations. As treatments for infectious diseases and the prevention of cardiovascular disease continues to improve, and the average life expectancy increases, cancer is likely to become the most common fatal disease in these countries. Therefore, successfully treating cancer requires that all the malignant cells be removed or destroyed without killing the patient. An ideal way to achieve this would be to induce an immune response against the tumour that would discriminate between the cells of the tumour and their normal cellular counterparts. However, immunological approaches to the treatment of cancer have been attempted for over a century with unsustainable results.

Accordingly, current methods of treating cancer continue to follow the long used protocol of surgical excision (if possible) followed by radiotherapy and/or chemotherapy, if necessary. The success rate of this rather crude form of treatment is extremely variable but generally decreases significantly as the tumour becomes more advanced and metastasises. Further, these treatments are associated with severe side effects including disfigurement and scarring from surgery (e.g. mastectomy or limb amputation), severe nausea and vomiting from chemotherapy, and most significantly, the damage to normal tissues such as the hair follicles, gut and bone marrow which is induced as a result of the relatively non-specific targeting mechanism of the toxic drugs which form part of most cancer treatments.

Further, most anti-cancer treatments, which include cytotoxic chemotherapeutic agents, signal transduction inhibitors, radiotherapy, monoclonal antibodies and cytotoxic lymphocytes, kill cancer cells by apoptosis. Although tumours may contain a proportion of apoptotic cells and even areas of necrosis before anti-cancer treatment is given, an increased number of apoptotic cells and larger areas of necrosis are anticipated in tumours that respond to the anti-cancer treatment. However, when cytotoxic chemotherapeutic agents are used for the treatment of advanced cancer, the degree of cell kill and thus the response of the tumour to the first treatment is frequently difficult to assess. Conventionally, patients receive a minimum of three cycles of chemotherapy before a clinical and radiological assessment of tumour response is made. Usually, only a minority of patients with advanced cancer responds to cytotoxic drugs and so patients may experience the side effects of treatment without obtaining benefit. Hence, there is an unmet medical need for a diagnostic method that would enable rapid, convenient and reliable detection of tumour cell kill after the first cycle of treatment that would predict treatment response, which in turn often predicts survival. For example, the use of positron emission tomography with fluoro-deoxyglucose (FDG-PET) in patients with oesophageal adenocarcinoma, who received chemoradiotherapy before surgery, differentiated treatment responders from non-responders with >90% sensitivity and specificity and tended to predict those who would subsequently undergo a curative resection of their tumours. Knowing whether the tumour is responding early would spare the majority of patients from ineffective and potentially toxic treatment. Then, non-responding patients can be offered second line treatments or clinical trials of investigational agents.

Accordingly, there is and urgent an ongoing need to develop new methods of diagnosing and treating cancers in a targeted manner. This notion of effective targeted killing of malignant cells has been, to date, unattainable.

In work leading up to the present invention it has been surprisingly determined that telomerase expression is upregulated in dead cancer cells, in particular cancer cells which have died as a result of an anti-cancer treatment. This finding is quite distinct from the prior art in which the use of telomerase as an indicator of the presence of cancer has been directed to its use as a diagnostic tool in the context of screening *live* cancer cells for telomerase activity. Accordingly, this determination has facilitated the development of a means for identifying dead and dying neoplastic cells based on screening for increased levels, relative to normal levels, of telomerase expression by dead cells. This is particularly important in the context of monitoring the progress of a therapeutic treatment regime which is directed to killing neoplastic cells. Still further, these findings have also enabled the use of telomerase as a means of facilitating the administration of anti-tumour therapy in a highly targeted and specific manner.

US 6,639,057 describes monoclonal hTERT antibodies, the use thereof in the detection and quantification of hTERT protein, and putative diagnostic methods and cancer therapies.

Kelland et al., 2001, Lancet Oncology 2(2): 95-102 describes the state of the art in relation to telomerase biology in the context of cancer diagnostics and therapeutics.

Hiyama et al., 2004, Cytotechnology 45(1-2): 61-74 describes that telomerase is activated in approximately 85% of human cancer tissues and that telomerase levels may be useful markers for cancer cell detection or as prognostic indicators, depending on the cancer type. The difficulty in detecting telomerase due to its low level of expression and mRNA instability is discussed. PCT-based methods for detection are suggested.

Sapi et al., 2002, Cancer Detection and Prevention 26(2): 158-167 describes the detection of circulating tumour cells in peripheral blood of ovarian cancer patients. Measurement of telomerase levels is performed in live ovarian cancer cells in peripheral blood.

Waguri et al., 2003, Clinical Cancer Research 9(8): 3004-3011 describes the detection of circulating cancer cell sin patients with hepatocellular carcinoma. Telomerase reverse transcriptase mRNA was detected in live cancer cells after immunomagnetic separation.

### SUMMARY OF THE INVENTION

Throughout this specification, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source.

The subject specification contains nucleotide sequence information prepared using the programme PatentIn Version 3.1, presented herein after the bibliography. Each nucleotide sequence is identified in the sequence listing by the numeric indicator <210> followed by the sequence identifier (eg. <210>1, <210>2, etc). The length, type of sequence (DNA, etc) and source organism for each sequence is indicated by information provided in the numeric indicator fields <211>, <212> and <213>, respectively. Nucleotide sequences referred to in the specification are identified by the indicator SEQ ID NO: followed by the sequence identifier (eg. SEQ ID NO:1, SEQ ID NO:2, etc.). The sequence identifier referred to in the specification correlates to the information provided in numeric indicator field <400> in the sequence listing, which is followed by the sequence identifier (eg. <400>1, <400>2, etc). That is SEQ ID NO:1 as detailed in the specification correlates to the sequence indicated as <400>1 in the sequence listing

Disclosed is a method for detecting non-viable neoplastic cells in a subject, said method comprising screening for the level of telomerase protein and/or gene expression by non-viable cells in said subject or in a biological sample derived from said subject wherein an increase in the level of non-viable cells expressing said telomerase relative to normal levels is indicative of the presence of non-viable neoplastic cells.

The present invention provides *an in vitro* method for detecting dead neoplastic cells in a subject undergoing an anti-neoplastic cell treatment regime, said method comprising screening for the level of telomerase RNA, mRNA or protein in dead cells which have lost membrane integrity in a biological sample derived from said subject wherein an increase in the level of telomerase in the dead cells relative to a control level of telomerase is indicative of the presence of dead neoplastic cells.

Yet another aspect of the present invention provides an *in vitro* method for detecting dead neoplastic cells in a subject undergoing an anti-neoplastic cell treatment regime, which cell death was induced by an anti-neoplastic cell treatment regime, said method comprising screening for the level of telomerase RNA, mRNA or protein in dead cells which have lost membrane integrity in a biological sample derived from said subject wherein an increase in the level of telomerase in the dead cells relative to a control level of telomerase is indicative of the presence of dead neoplastic cells.

Another aspect of the present invention provides an *in vitro* method for detecting dead neoplastic cells in a subject undergoing an anti-neoplastic cell treatment regime, said method comprising screening for the level of hTERT protein or mRNA or fragment, mutant or variant thereof in dead cells which have lost membrane integrity in a biological sample derived from said subject wherein an increase in the level of hTERT in the dead cells relative to a control level of hTERT is indicative of the presence of dead neoplastic cells.

Yet another aspect of the present invention provides an *in vitro* method for detecting dead neoplastic cells in a subject undergoing an anti-neoplastic cell treatment regime, said method comprising screening for the level of hTR RNA or fragment, mutant or variant thereof in dead cells which have lost membrane integrity in a biological sample derived from said subject wherein an increase in the level of hTR RNA in the dead cells relative to a control level of hTR RNA is indicative of the presence of dead neoplastic cells.

Disclosed is a method for assessing and/or monitoring a neoplastic condition in a subject, said method comprising screening for the level of telomerase protein and/or gene expression by non-viable cells in said subject or in a biological sample derived from said subject wherein an increase in the level of non-viable cells expressing said telomerase relative to normal levels is indicative of the presence of non-viable neoplastic cells. Yet still another aspect of the present invention provides an *in vitro* method for assessing and/or monitoring a neoplastic condition in a subject undergoing an anti-neoplastic cell treatment regime, said method comprising screening for the level of telomerase RNA, mRNA or protein in dead cells which have lost membrane integrity in a biological sample derived from said subject wherein an increase in the level of telomerase in the dead cells relative to a control level of telomerase is indicative of the presence of dead neoplastic cells.

A further aspect of the present invention provides *an in vitro* method for assessing and/or monitoring a neoplastic condition in a subject undergoing an anti-neoplastic cell treatment regime, said method comprising screening for the level of hTERT protein or mRNA or fragment, mutant or variant thereof in dead cells which have lost membrane integrity in a biological sample derived from said subject wherein an increase in the level of hTERT in the dead cells relative to a control level of hTERT is indicative of the presence of dead neoplastic cells.

Another further aspect of the present invention provides an *in vitro* method for assessing and/or monitoring a neoplastic condition in a subject undergoing an anti-neoplastic cell-treatment regime, said method comprising screening for the level of hTR RNA or fragment, mutant or variant thereof in dead cells which have lost membrane integrity in a biological sample derived from said subject wherein an increase in the level of hTR RNA in the dead cells relative to a control level of hTR RNA is indicative of the presence of dead neoplastic cells.

Disclosed is assessing and/or monitoring the effectiveness of a neoplasm therapeutic treatment regime in a subject said method comprising screening for the level of telomerase protein and/or gene expression by non-viable cells in said subject or in a biological sample derived from said subject wherein an increase in the level of non-viable cells expressing said telomerase relative to normal levels is indicative of the induction of neoplastic cell death.

Disclosed is a diagnostic kit for a biological sample comprising in compartmental form a first compartment adapted to contain an agent for detecting telomerase or a nucleic acid molecule encoding telomerase and a second compartment adapted to contain reagents useful for facilitating the detection by the agent in the first compartment. Further compartments may also be included, for example, to receive a biological sample. The agent may be an antibody or other suitable detection molecule.

Disclosed is the use of an interactive molecule directed to telomerase in the manufacture of a quantitative or semi-quantitative diagnostic kit to detect dead neoplastic cells in a biological sample from a patient. The kit may come with instructions for use and may be automated or semi-automated or in a form which is compatible with an automated machine or software.

Disclosed is a method of treating a neoplastic condition in a subject said method comprising administering to said subject an effective amount of an interactive molecule directed to telomerase or antigenic portion thereof, which interactive molecule is linked, bound or otherwise associated with an effector mechanism, for a time and under conditions sufficient to treat said condition.

Disclosed is a method of treating a neoplastic condition in a subject, said method comprising administering to said subject an effective amount of an immunointeractive molecule directed to hTERT protein or antigenic portion thereof, which immunointeractive molecule is linked, bound or otherwise associated with an effector mechanism, for a time and under conditions sufficient to inhibit, reduce or otherwise down-regulate the growth of the neoplasm.

Disclosed is a method of treating a neoplastic condition in a subject, said method comprising administering to said subject an effective amount of an immunointeractive molecule directed to hTERT or hTR RNA or antigenic portion thereof, which immunointeractive molecule is linked, bound or otherwise associated with an effector mechanism, for a time and under conditions sufficient to inhibit, reduce or otherwise down-regulate the growth of the neoplasm.

Disclosed is a method of therapeutically treating a metastatic cancer in a subject, said method comprising administering to said subject an effective amount of an interactive molecule directed to telomerase or antigenic portion thereof, which interactive molecule is linked, bound or otherwise associated with a therapeutic effector mechanism, for a time and under conditions sufficient to inhibit, reduce or otherwise down-regulate the growth of said metastatic cancer.

Disclosed is an anti-telomerase interactive molecule conjugated to an effector mechanism, in the manufacture of medicament for the treatment of a neoplastic condition in a subject wherein said effector mechanism treats said condition.

Disclosed is a pharmaceutical composition comprising the modulatory agent as hereinbefore defined together with one or more pharmaceutically acceptable carriers and/or diluents.

Disclosed is the agent as hereinbefore defined, when used in the method of the present invention.

Also disclosed are the above methods in which the screening is performed in said subject.

The present invention also provides an anti-telomerase immuno-interactive molecule or imaging probe capable of disclosing altered levels of the telomerase RNA, mRNA or protein expression product for use in an in vivo method of detecting dead neoplastic cells in a subject undergoing an anti-neoplastic cell treatment regime, said method comprising using said molecule or probe to screen for the level of telomerase expression in dead cells which have lost membrane integrity in said subject, wherein an increase in the level of telomerase in the dead cells relative to a control level of telomerase is indicative of the presence of dead neoplastic cells.

The present invention also provides an anti-telomerase immune-interactive molecule or imaging probe capable of disclosing altered levels of the telomerase RNA, mRNA or protein expression product for use in an in vivo method of assessing or monitoring a neoplastic condition in a subject undergoing an anti-neoplastic cell treatment regime, said method comprising using said molecule or probe to screen for the level of telomerase expression in dead cells which have lost membrane integrity in said subject, wherein an increase in the level of telomerase in the dead cells relative to a control level of telomerase is indicative of the presence of dead neoplastic cells.

### BRIEF DESCRIPTION OF THE DRAWINGS

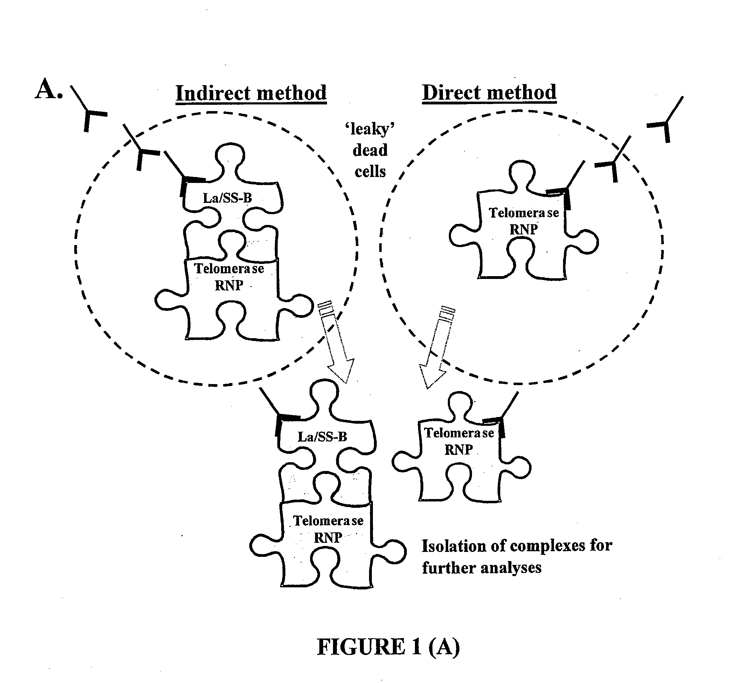

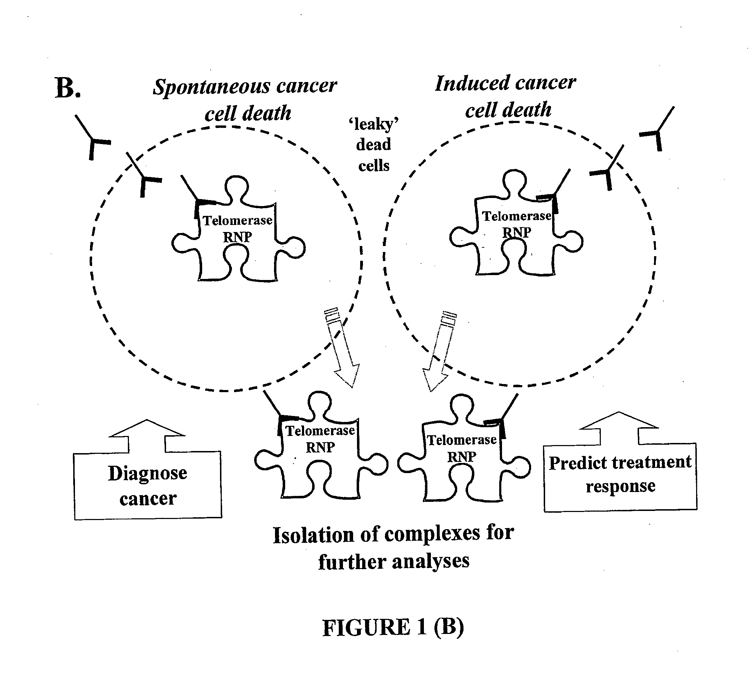

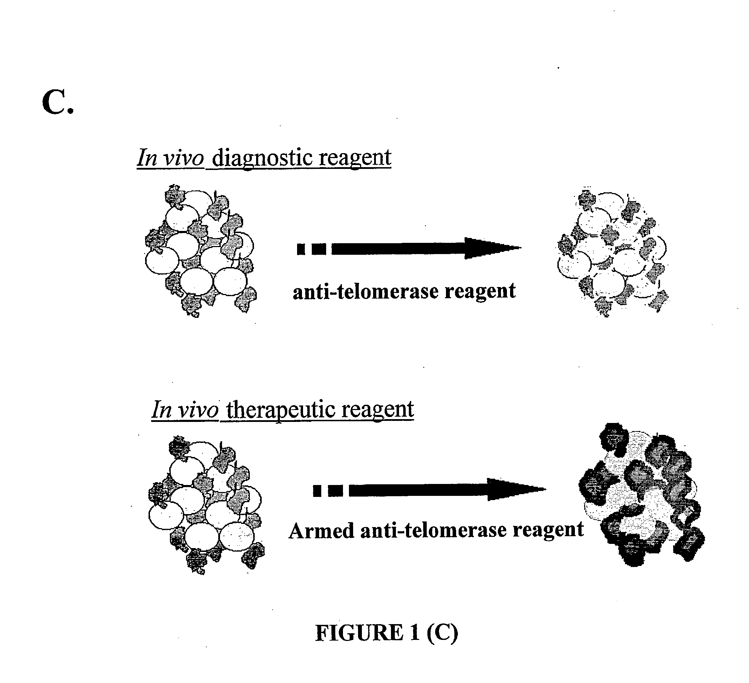

**Figure 1** is a schematic representation of the *in vitro* and *in vivo* diagnostic and therapeutic uses to which telomerase interactive molecules may be put. A. *Via* the binding of the La protein to the telomerase ribonucleoprotein (RNP), La interactive molecules may be used in an indirect method to identify components of the telomerase RNP, which include the integral RNA (hTR) together with the catalytic component (hTERT). Telomerase interactive molecules *per se* may be used in a direct method to detect hTR and hTERT. **B.** In the case of cancer cell death that is either spontaneous or induced by anti-cancer treatment, telomerase interactive molecules *may be used specifically to diagnose the presence of cancers or to predict response to anti-cancer treatment.* **C.** After the first chemotherapy, an anti-telomerase reagent may have utility as an in vivo diagnostic reagent via the detection of dead cancer cells (dark grey), which lie close to viable cancer cells (light grey). Similarly, a telomerase interactive molecule may be useful as an in vivo therapeutic reagent if it were armed with a non-cross resistant anti-cancer treatment (purple) that would deliver bystander killing to the remaining live cancer cells (red).

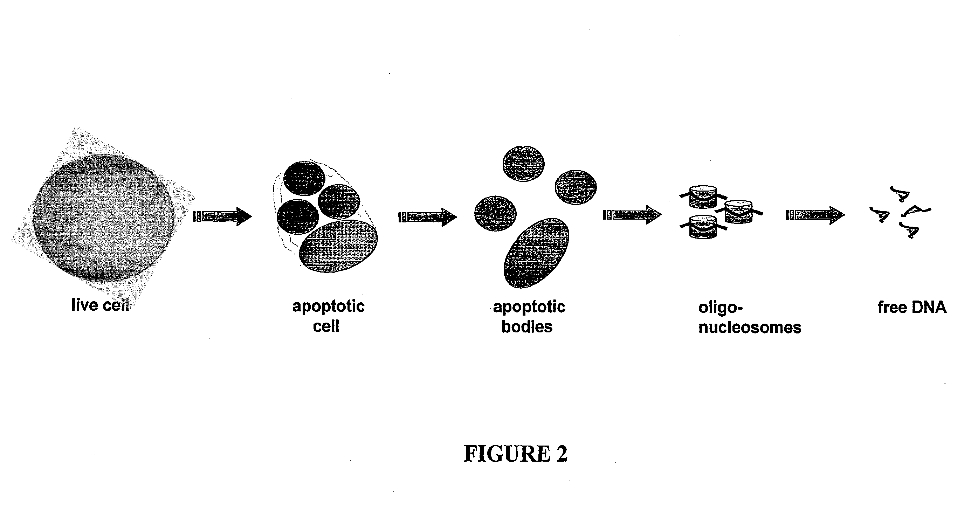

**Figure 2** is a schematic representation of the progression of apoptosis through various states *in vitro.* After an apoptotic stimulus, apoptotic cells shrink and fragment into membrane bound parcels known as apoptotic bodies that become increasingly leaky or secondarily necrotic with time. Eventually the apoptotic bodies disintegrate to oligonucleosomes and then free DNA.

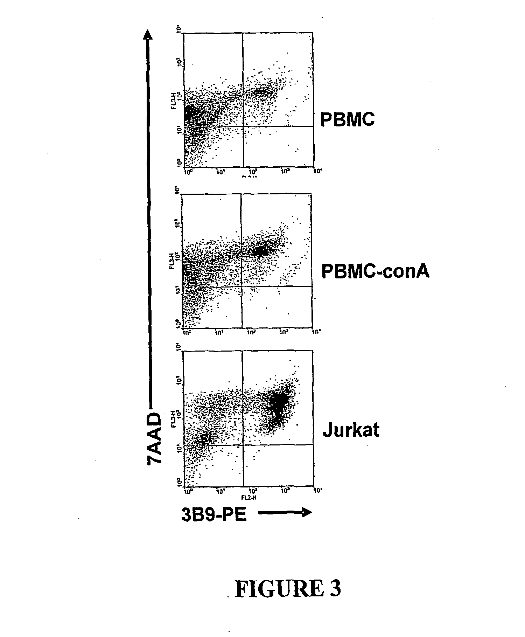

**Figure 3** is a graphical representation of the up-regulation of La/SS-B expression after apoptosis of malignant Jurkat T cells in comparison with apoptotic primary T cells. Ficoll-purified peripheral blood mononuclear cells (PBMC) were cultured for 4d in RPMI-1640 with 10% fetal calf serum and then treated with 1µM staurosporine in the final 24h of culture. Similarly, PBMC were activated with the T cell mitogen conconavalin and then apoptosis was induced using 1µM staurosporine for 24h. Jurkat cells (Jurkat) were rendered apoptotic by 24h treatment with 0.5µM staurosporine. Quadrant cursors are set for <3% staining with isotype control, Sal5.

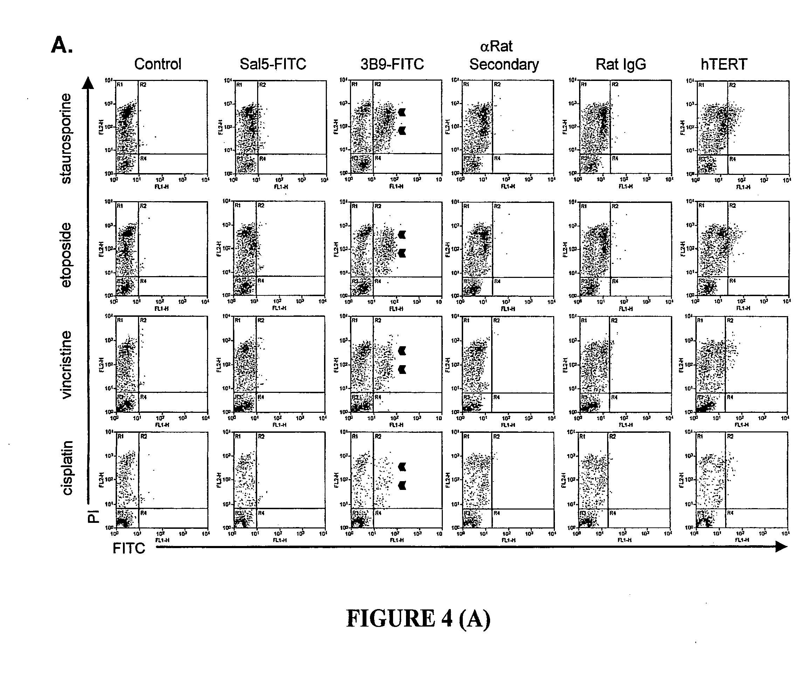

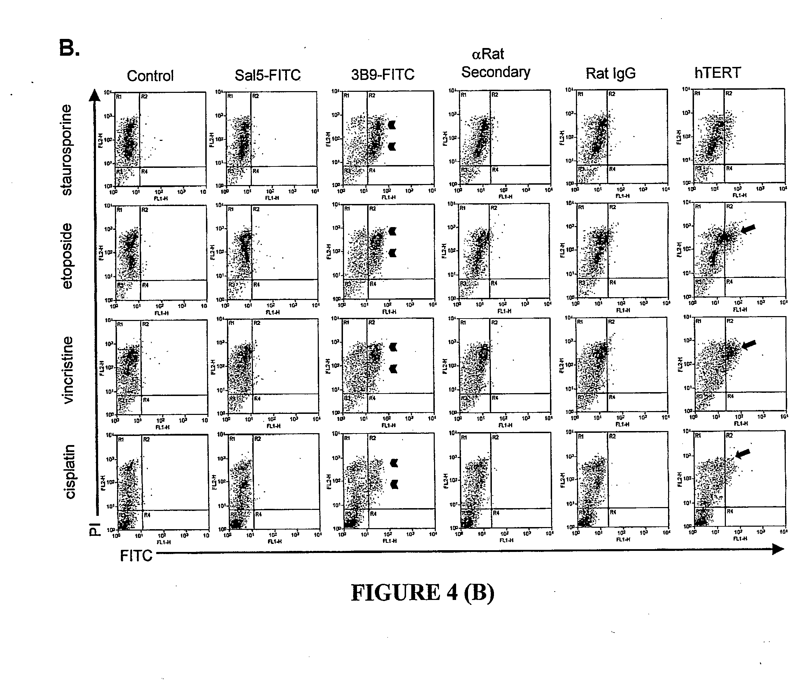

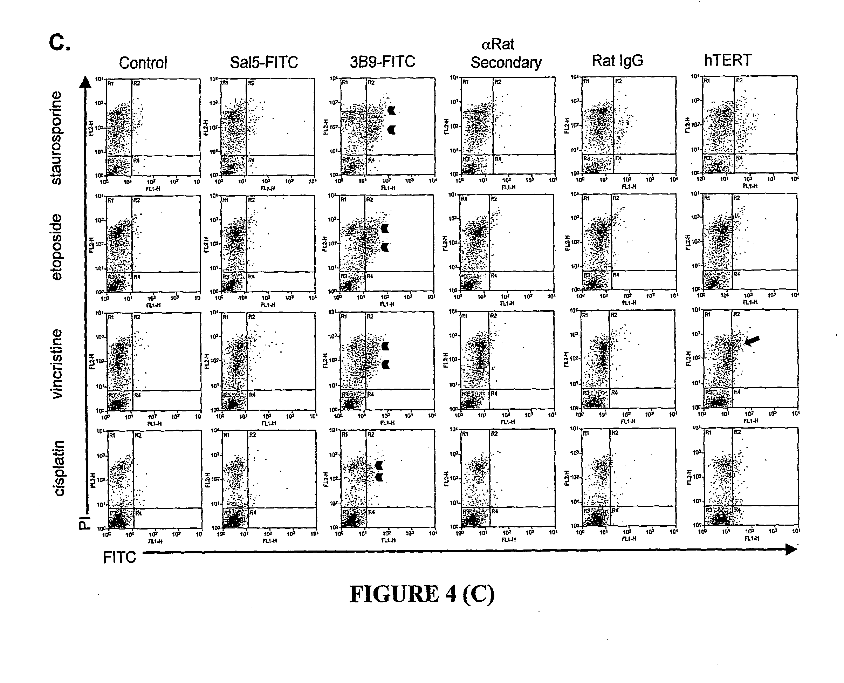

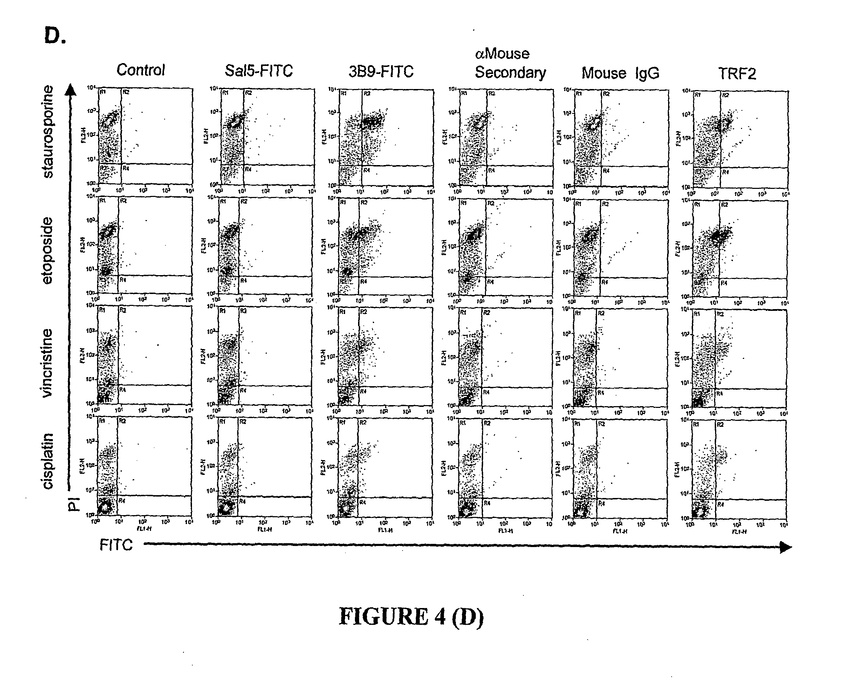

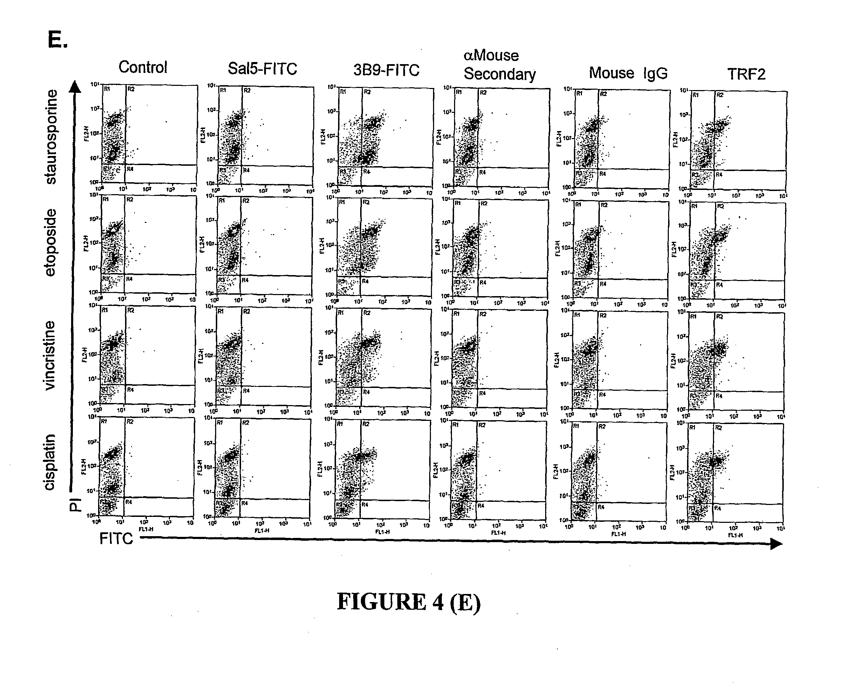

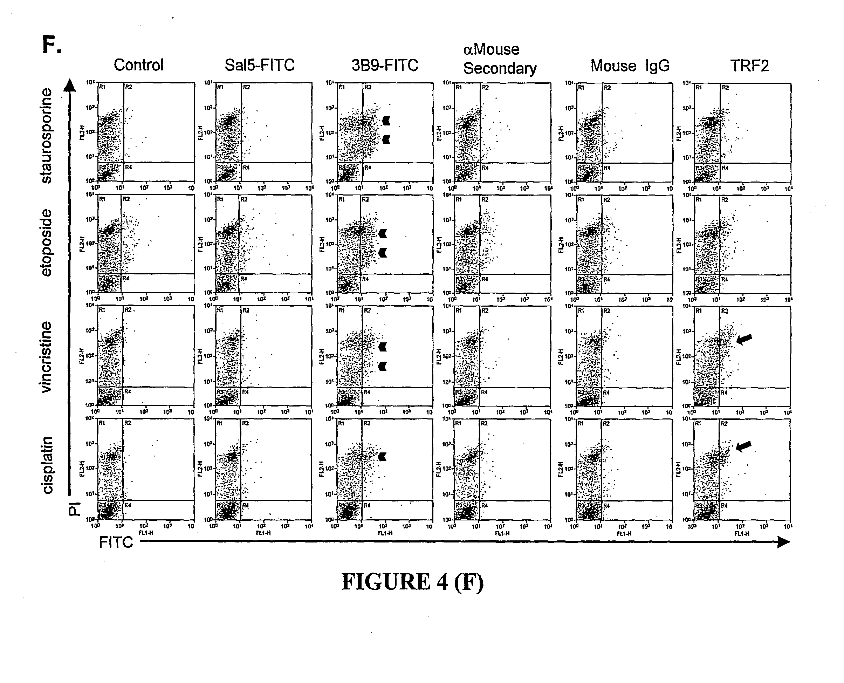

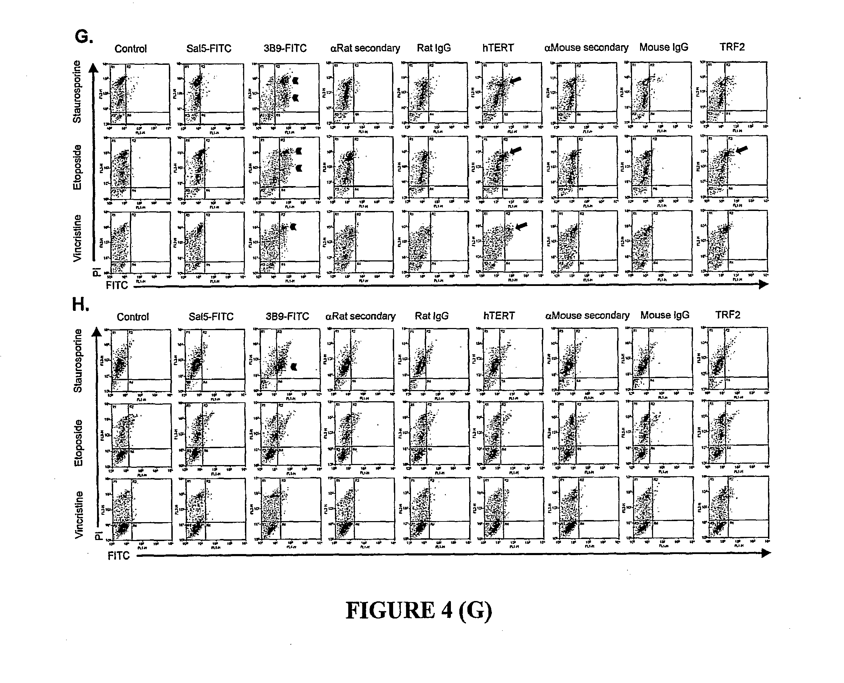

**Figure 4** is a graphical representation of the cytotoxic treatment of malignant human cells results in increased expression of TRF2 and La in apoptotic cells 24h after treatment and in increased expression of hTERT in apoptotic cells 48h after treatment. **A. J**urkat cells after 24h treatment with cytotoxic agents. While La staining was evident in PI^{high} and PI^{intermediate} cells 24h after treatment with all cytotoxic agents (chevrons), no staining for hTERT was evident. **B. J**urkat cells after 48h treatment with cytotoxic agents. While La staining was evident in PI^{high} and PI^{intermediate} cells 48h after treatment with all cytotoxic agents (chevrons), only 48h treatment with etoposide, vincristine and cisplatin produced increased staining for hTERT and only in PI^{high} cells (arrows). **C. R**aji cells after 24h treatment with cytotoxic agents. While La staining was evident in PI^{high} and PI^{intermediate} cells 24h after treatment with all cytotoxic agents (chevrons), marginally increased staining for hTERT was observed after 24h treatment with vincristine (arrow). **D. J**urkat cells after 24h treatment with cytotoxic agents. Both La (3B9-FITC) and TRF2 staining were observed in apoptotic cells after treatment with all cytotoxic agents. **E. J**urkat cells after 48h treatment with cytotoxic agents. Staining for La (3B9-FITC) and TRF2 in apoptotic cells was more evident 48h after treatment with all cytotoxic agents. **F. R**aji cells after 48h treatment with cytotoxic agents. Although La staining was evident in apoptotic cells 48h after treatment with all cytotoxic agents (chevrons), only cells treated with vincristine and cisplatin demonstrated increased TRF2 staining. **G. H**eLa cells after 48h treatment with cytotoxic agents. Staurosporine, etoposide and vincristine induced apoptosis and La staining (chevrons) together with staining for hTERT but little staining for TRF2 (arrows) H. U2OS cells after 48h treatment with cytotoxic agents. Staurosporine only induced apoptosis and La staining (chevron) but there was no corresponding staining for hTERT or TRF2. In each case, the staining is indicated above each column of panels and the cytotoxic employed is indicated to the left of each row of panels. Also indicated is a PI only control and negative controls for hTERT staining (purified rat IgG or anti-rat secondary antibody alone) and for TRF2 staining (purified mouse IgG or anti-mouse secondary antibody alone).

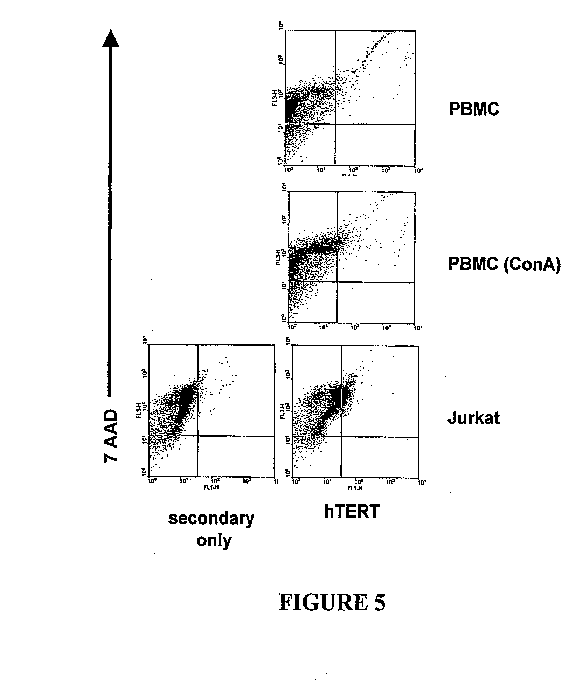

**Figure 5** is a graphical representation of the culturing of PMBC with conconavalin A (conA) 10µg/mL for 72h. Viable cells were obtained by density gradient centrifugation and then apoptosis was induced using 1µM staurosporine for 24h. Jurkat cells were also treated with 1µM staurosporine for 24h. Dot plots depict staining for 7-AAD and hTERT. The negative control is staining with the secondary antibody alone.

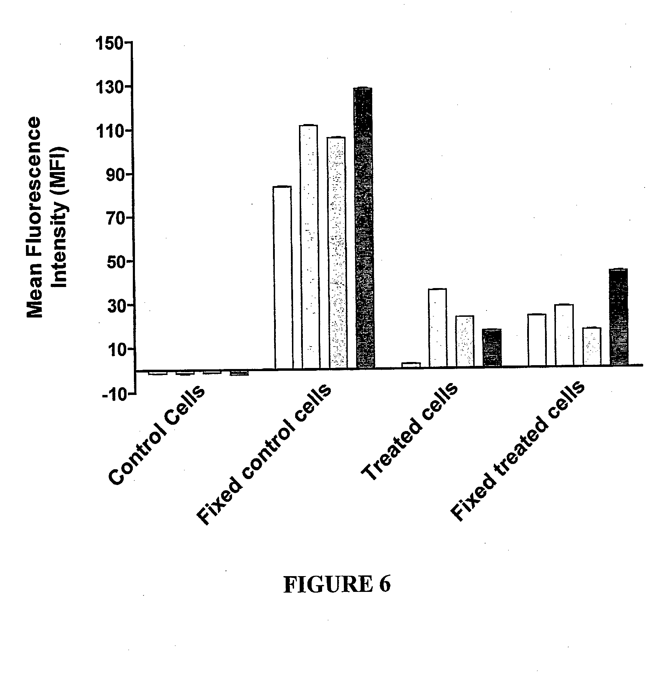

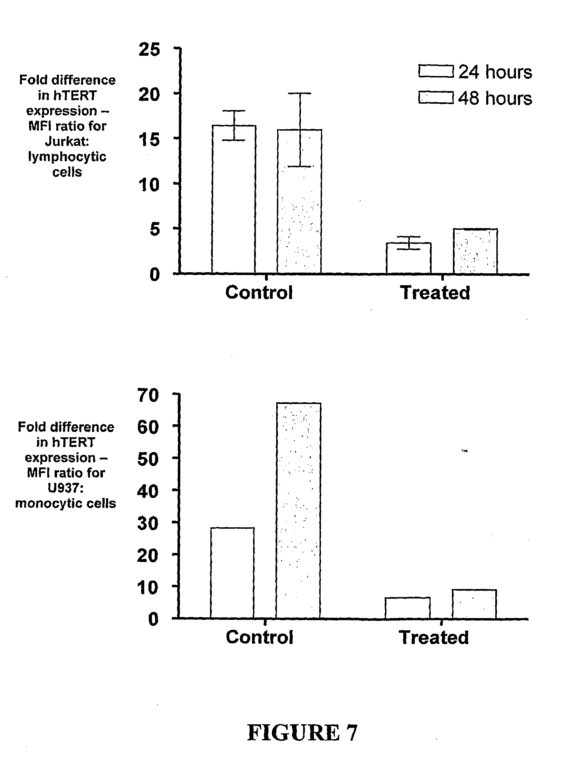

**Figure 6** is a graphical representation of telomerase detection over time in untreated (control) and etoposide-treated Jurkat cells. Cells were cultured in the absence (control) or presence (treated) of etoposide 200 µg/mL for 24h (clear bars), 48h (light grey bars), 72h (dark grey bars) and 96h (black bars). Cells were collected at the specific time points were incubated at 5 x 10⁶ cells/mL for 10 minutes at room temperature in either in PBS (unfixed) or 2% paraformaldehyde solution (fixed). The fixed cells were then diluted 1:10 with -20°C methanol for 5 minutes. All cells were then double stained at a concentration of 50µg/mL as described in Figure 7 and the mean fluorescence intensity of dead (7-AAD⁺) cells in the FL2 channel (PE) was plotted for the different conditions (*n*=2). Standard error of the mean is plotted but is too small to be visible on the graph.
**Figure 7** is a graphical representation of telomerase detection in the malignant counterparts of normal primary blood cells. Peripheral blood mononuclear cells (PBMC) that were cultured overnight could be separated into two major sub-populations because they either adhered to the plastic tissue culture dish (predominantly as monocytic cells) or remained in suspension (predominantly as T lymphocytes). The malignant counterparts of monocytic cells are U937 cells and of T lymphocytes are Jurkat cells. The expression of hTERT protein was compared in transformed cells compared to primary cells. To induce apoptosis, the malignant cells (U937 or Jurkat cells) and primary cells (adherent monocytic or suspension lymphocytic blood cells) were incubated in RPMI-1640 with 5% FCS in the presence or absence of staurosporine (STS) for 24h (clear bars) and 48h (grey bars). The untreated (control) cells were permeabilised as described in the legend of Figure 6. The permeabilised control cells and unprocessed STS-treated cells were incubated in 50µg/mL of anti-hTERT-biotin for 30 min. at room temperature. Cells were washed with PBS then incubated for 30 min. at room temperature with 1:100 dilution of streptavidin-PE. Cells were washed with PBS and incubated in 2µg/mL 7-AAD solution for 10 minutes then analysed using flow cytometry. The fold difference in hTERT expression is shown and derives from the ratio of mean fluorescence intensities (MFI) of hTERT expression for Jurkat and U937 cells to that for the primary suspension lymphocytic and adherent monocytic cells, respectively. For the upper panel, measurements were performed in duplicate and SEM is shown. For the lower panel, a single measurement was made that is representative of two independent experiments.

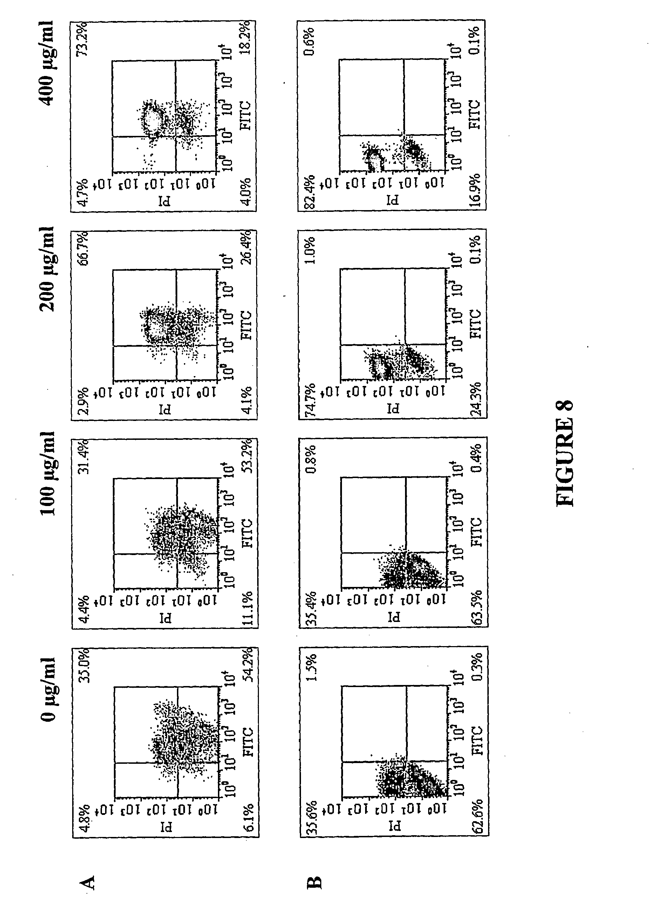

**Figure 8** is a graphical representation of the staining of H69 cells using the anti-epithelial marker monoclonal antibody BerEP4-FITC. H69 cells (1 x 10⁶ cells/mL) were cultured in RPMI-1640 containing 5% FCS in the absence or presence of the specified concentrations of etoposide (0-400µg/mL). After 48 hours of incubation, cells were collected and incubated at room temperature for 15 minutes with (A) 5µg/mL BerEP4-FITC or (B) 5µg/mL mouse IgG_{1aκ}-FITC isotype. Cells were washed and pellets were resuspended in 0.5µg/mL solution of PI in PBS then analysed by flow cytometry.

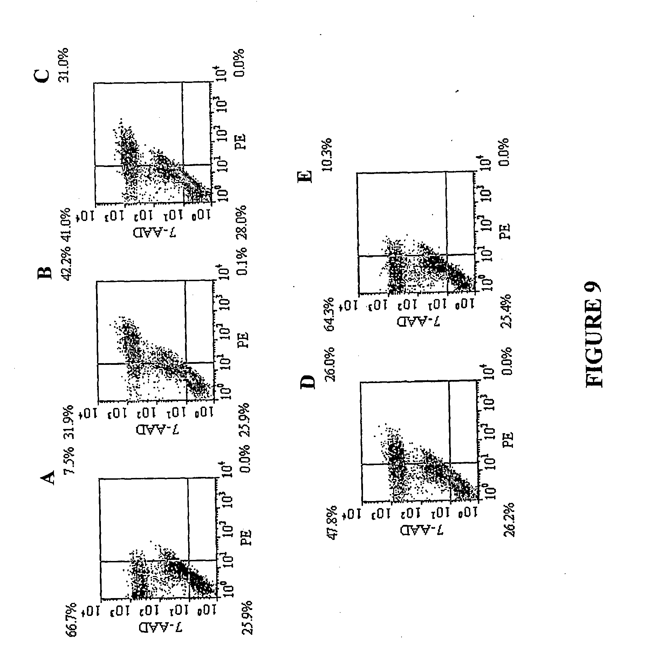

**Figure 9** is a graphical representation of detection of hTERT protein in etoposide-treated H69 cells. H69 cells were cultured for 48 hours in the presence of 200µg/mL etoposide. Cells were collected, washed and incubated for 30 minutes at room temperature with **(A)** 50µg/mL rat IgG_{2aκ}-biotin or **(B)** 50µg/mL, **(C)** 25µg/mL, **(D)** 12.5µg/mL or **(E)** 2.5µg/mL of rat anti-human telomerase biotin-conjugated monoclonal antibody. Cells were washed and incubated with 1:100 dilution of streptavidin-PE for 30 minutes at room temperature. Cells were washed, incubated with 2µg/mL 7-AAD for 10 minutes at room temperature then analysed by flow cytometry.

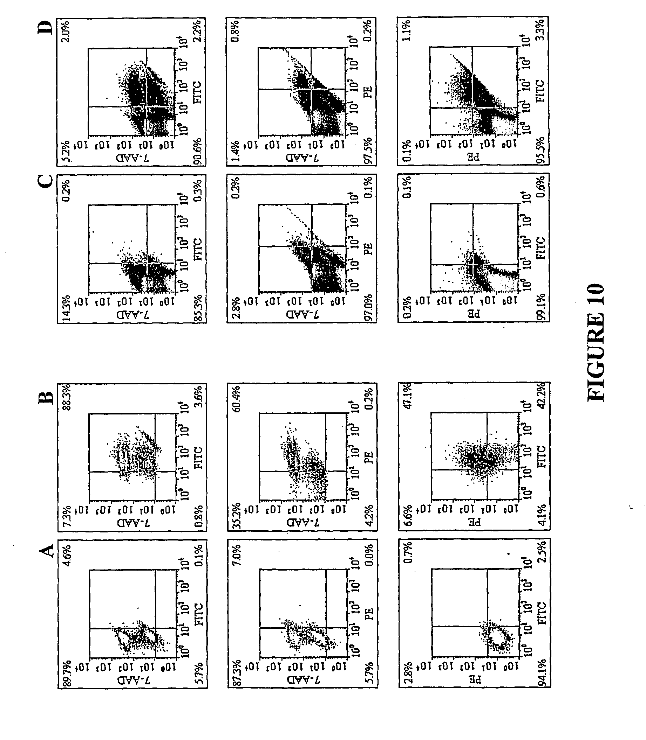

**Figure 10** is a graphical representation of triple colour staining of etoposide-treated H69 cells. H69 cells cultured for 48 hours in the presence of 400µg/mL etoposide were collected and incubated in the presence of **(A, C)** 5µg/mL mouse IgG_{1aκ}-FITC and 50µg/mL rat IgG_{2aκ}-biotin or **(B, D)** 5µg/mL BerEP4-FITC and 50µg/mL anti-telomerase-biotin. Cells were washed and incubated for 30 minutes at room temperature in 1:100 dilution of streptavidin-PE. Cells were washed and incubated with 2µg/mL 7-AAD for 10 minutes at room temperature. Cells were analysed directly **(A, B)** or first diluted 1:50 in peripheral blood mononuclear cells **(C, D)** and then analysed by flow cytometry.

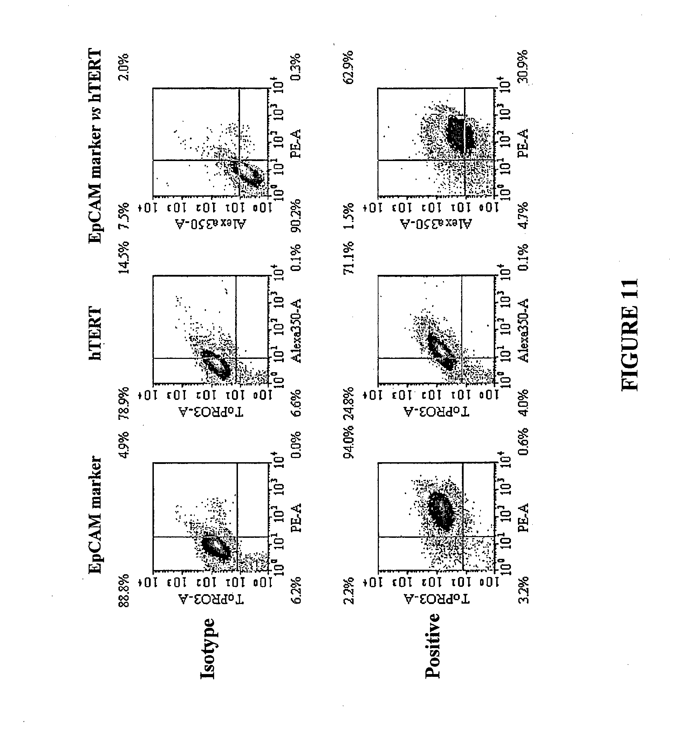

**Figure 11** is a graphical representation of triple-colour staining of etoposide-treated H69 cells using a more sensitive technique. Etoposide-treated H69 cells were incubated for 15 min. at room temperature with 10µg/mL of mouse IgG_{1aκ}-PE and 50µg/mL rat IgG₂ₐκ-Alexa350 as isotype controls or 10µg/mL BerEP4-PE and 50µg/mL anti-hTERT-Alexa350. Cells were washed with PBS and incubated with 1mg/mL TOPRO-3 DNA dye for 10 min. at room temperature before analysis using flow cytometry (LSR-II system).

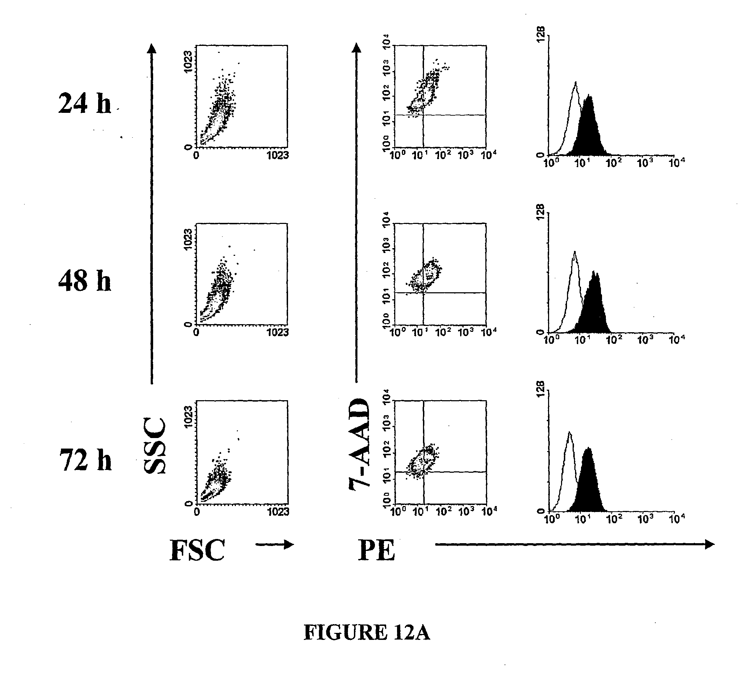

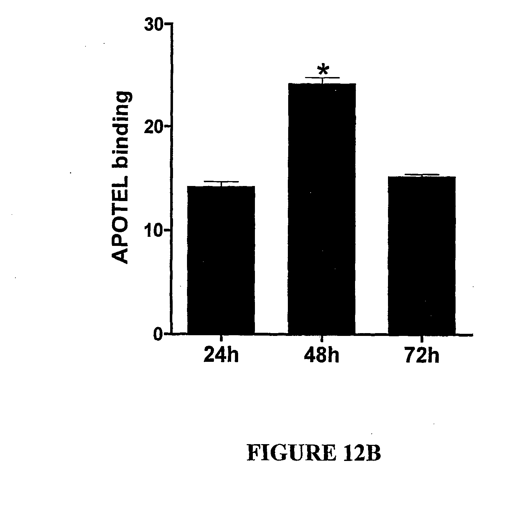

**Figure 12** is a representation of APOTEL™ specifically binding *in vitro* to cells of the H69 small cell lung cancer cell line (SCLC), which have been rendered apoptotic by cytotoxic drug treatment. Etoposide-treated H69 cells were analyzed by flow cytometry 24h, 48h and 72h after induction of cytotoxic treatment. **(A)** Shown are density plots of forward scatter (FSC) *vs.* side scatter (SSC) (left-hand column of panels), 7-AAD fluorescence *vs.* PE fluorescence (middle column of panels) and histogram overlay plots of PE fluorescence of 7-AAD-gated events (right-hand column of panels). Quadrants in the 7-AAD *vs.* PE density plots were drawn to indicate where cells were viable (7-AAD-negative) and where ≤3% of cells were positive for the isotype control. Histograms show PE fluorescence frequency distributions for binding with isotype control (open) and APOTEL™ (filled). **(B)** Column graph depicts specific APOTEL™ binding, which is shown as the net mean fluorescence intensity (MFI) calculated as the differences in MFI of binding of APOTEL™ and isotype control ± standard error of the mean (SEM, *n* = 3). *, P<0.001 for one-way analysis of variance (ANOVA) using Tukey's comparison of all pairs.

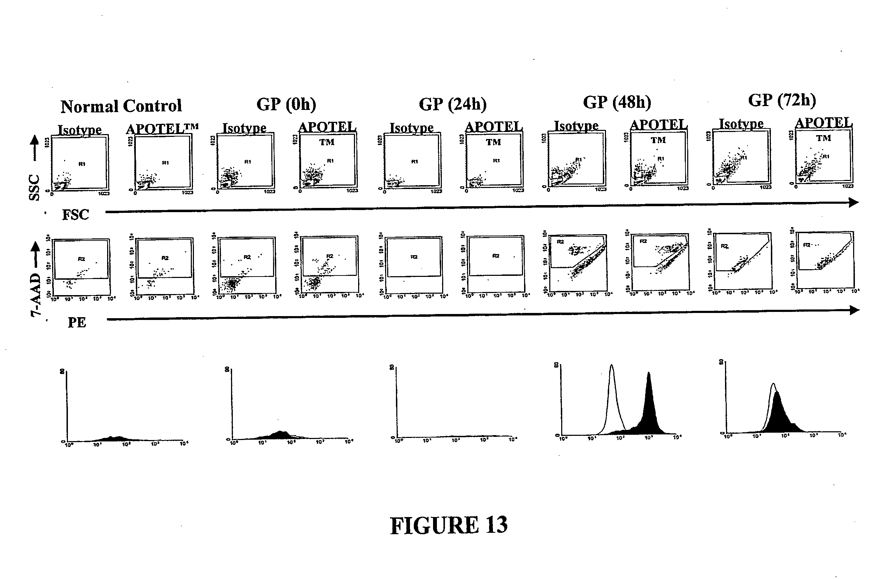

**Figure 13****:** The blood of a small cell lung cancer (SCLC) patient contains EpCAM-expressing tumour cells, and after the patient was treated with cytotoxic chemotherapy, the circulating tumour cells lost viability and bound APOTEL™ 48h post-treatment. Peripheral blood samples from a normal healthy volunteer (normal control; NC) or a patient (GP) who had extensive-stage SCLC were enriched for the expression of epithelial cell adhesion molecule (EpCAM) and analysed by flow cytometry. FSC *vs.* SSC density plots were constructed for all samples (upper row of panels). A region (R1) was drawn based on analysis of control blood to exclude any events related to red blood cells in particular. Patient (GP) samples were gated on the R1 region and are represented as density plots of 7-AAD *vs.* PE fluorescence (middle row of panels). A second region (R2) was constructed to select dead (7-AAD+) cells. PE-fluorescence for populations R1- and R2-gated are presented as histogram overlays for samples stained with APOTEL™ (filled) or matching isotype control mAb (open) (lower row of panels).
   *Notes:* (i) Bead separation appeared to fail at the 24h time point. (ii) In the case of both control and test samples at the 48 and 72 h time points, we observed an identical and highly fluorescent subpopulation of events, which was suspected to result from autofluorescence of beads associated non-specifically with dead cells or dead cell fragments. The R2 region for those samples was adjusted to exclude this subpopulation.

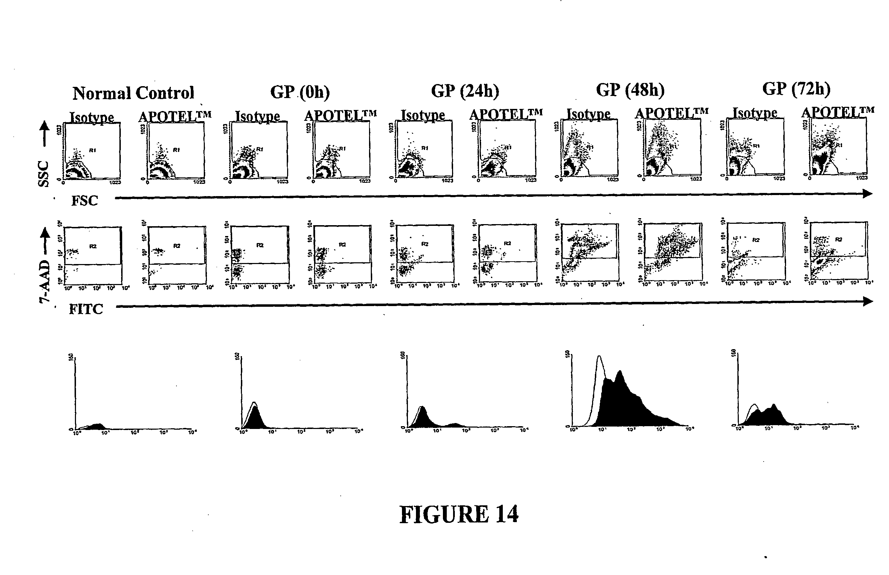

**Figure 14** is a graphical representation depicting that after treatment of a SCLC patient with cytotoxic chemotherapy, peak numbers of dead cells were found in the patient's blood 48h post-treatment when APOTEL™ binding was also maximal. Peripheral blood samples from a normal healthy volunteer (normal control; NC) or a patient (GP) who had extensive-stage SCLC were subject to red cell lysis analysed by flow cytometry. FSC *vs*. SSC density plots were constructed for all samples (upper row of panels). A region (R1) was drawn based on analysis of control blood to exclude any events related to red blood cells in particular. Patient (GP) samples were gated on the R1 region and are represented as density plots of 7-AAD *vs.* FITC fluorescence (middle row of panels). A second region (R2) was constructed to select dead (7-AAD⁺) cells. FITC-fluorescence for populations R1- and R2-gated are presented as histogram overlays for samples stained with APOTEL™ (filled) or matching isotype control mAb (open) (lower row of panels).
   *Note:* As evidenced from the appearance of 7-AAD⁺ events in the blood of the normal control, the red cell lysis step has contributed to an increase in the number of dead cells recorded at the 0h time point.

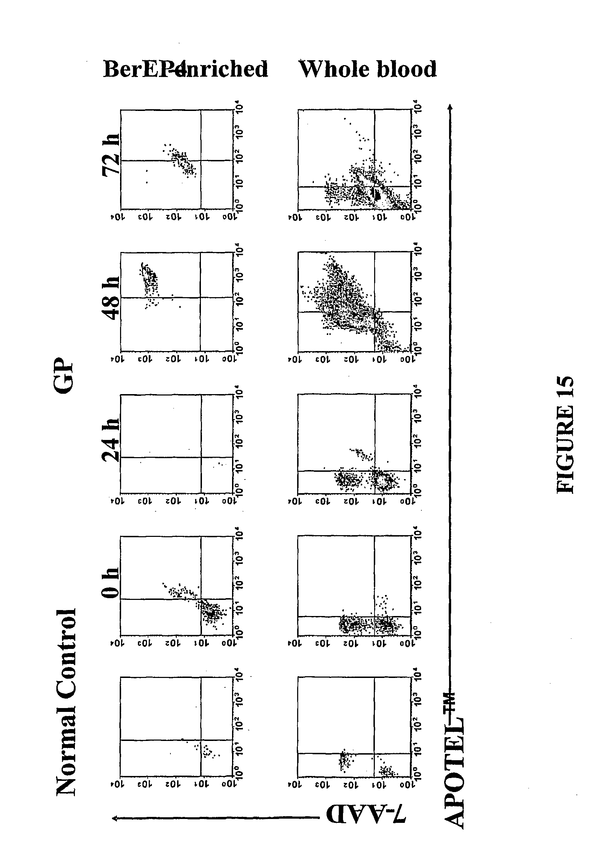

**Figure 15** is a graphical representation of the comparison of APOTEL™ binding in BerEP4-enriched and whole blood samples from a SCLC patient (GP) before and after treatment with cytotoxic chemotherapy. Shown are R1- and R2-gated density plots for 7-AAD *vs*. *A*POTEL™ for the samples from the normal control (NC) and the patient (GP) Quadrants in these plots were drawn to indicate where cells were viable (7-AAD-negative) and where ≤3% of cells were positive for the isotype control.

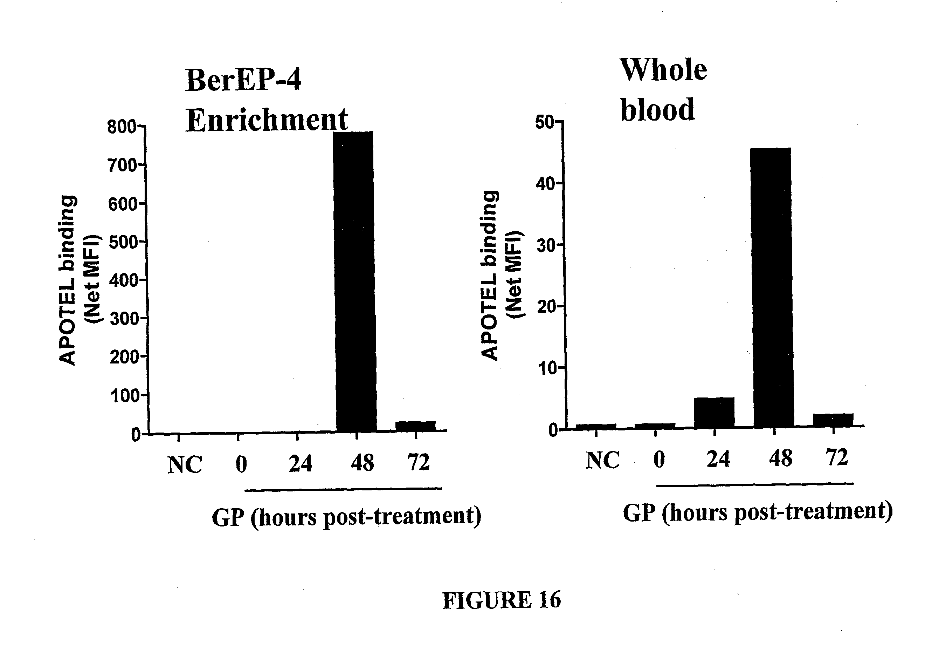

**Figure 16** is a graphical representation depicting that APOTEL™ binding to peripheral blood cells peaks 48h after treatment of a SCLC patient with cytotoxic chemotherapy. Column graph depicts specific APOTEL™ binding, which is shown as the net mean fluorescence intensity (MFI) calculated as the differences in MFI of binding of APOTEL™ and isotype control to the subpopulations, which were gated on R1 and R2 as described in Figures 2 and 3.

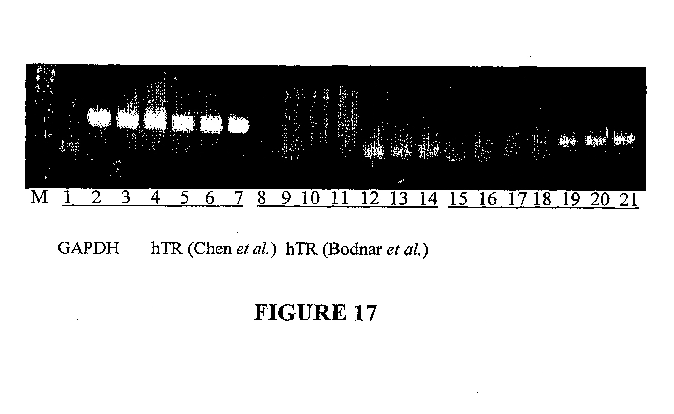

**Figure 17** is an image of RT-PCR of GAPDH and hTR mRNA from hTR-positive Jurkat cells and hTR-negative U2OS cells. M, molecular weight marker; Lanes 1, 8, 15: negative controls; Lanes 2, 9, 16: cDNA of apoptotic U2OS cells at 24h; Lanes 3, 10, 17: cDNA of apoptotic U2OS cells at 48h; Lanes 4, 11, 18: cDNA of apoptotic U2OS cells at 72h; Lanes 5, 12, 19: cDNA of apoptotic Jurkat cells at 24h; Lanes 6, 13, 20: cDNA of apoptotic Jurkat cells at 48h; Lanes 7, 14, 21: cDNA of apoptotic Jurkat cells at 72h.

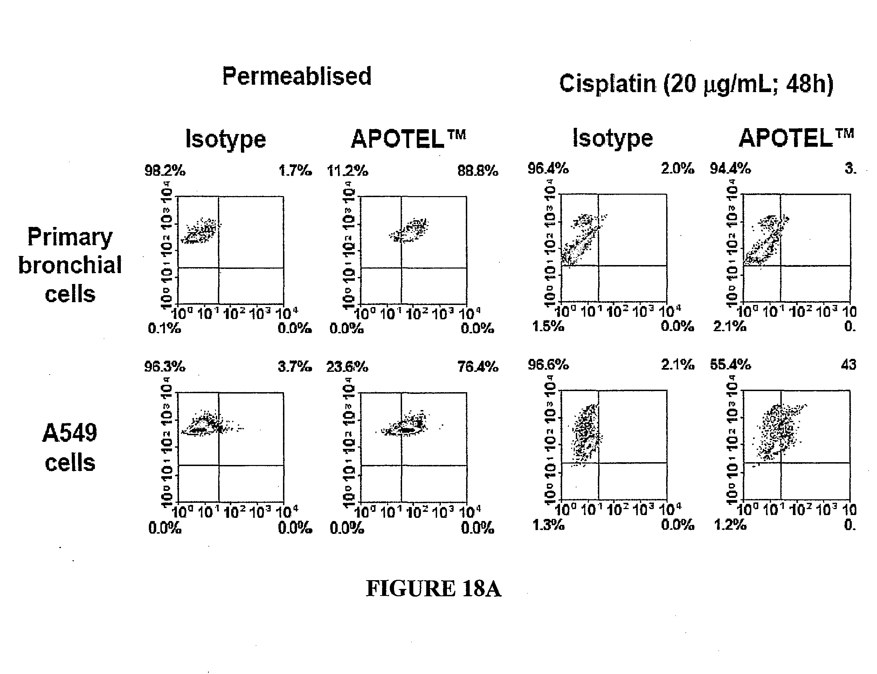

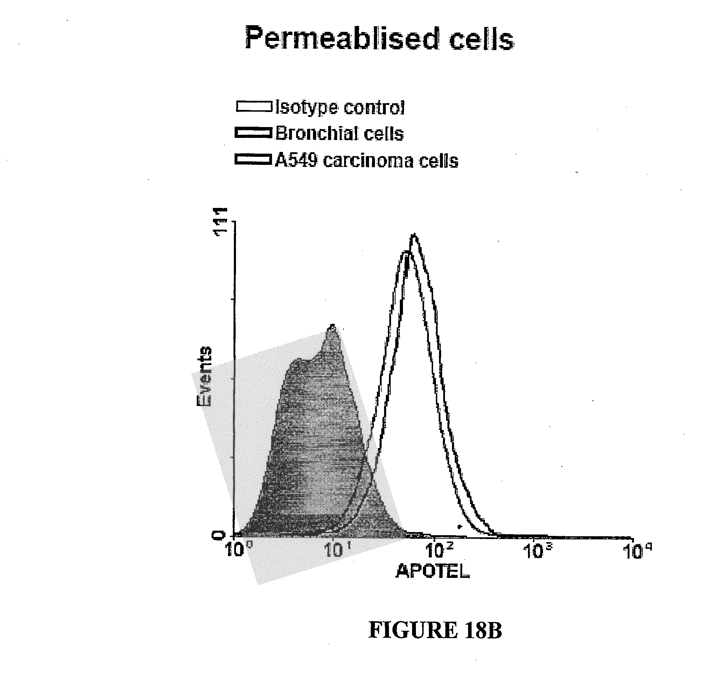

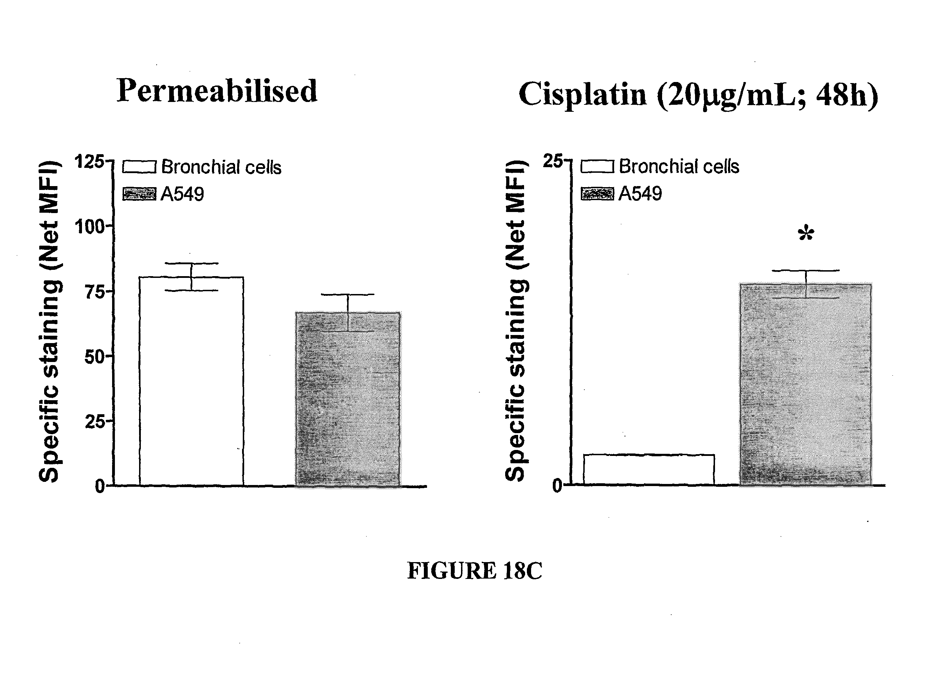

**Figure 18****:** Telomerase expression is equivalent in permeabilised primary and malignant bronchial epithelial cells but is increased significantly in bronchial carcinoma cells after cytotoxic drug treatment. **(A)** Density plots showing 7-AAD fluorescence (*y*-axis) *vs.* Alexa488 fluorescence (*x*-axis) for permeabilised and cisplatin-treated primary and malignant bronchial epithelial cells. Quadrants are set where cells were viable (7-AAD-negative) and where staining with the isotype control was <3%). **(B)** Histograms show frequency distributions for Alexa488 fluorescence on 7-AAD-gated events. **(C)** Column graphs show hTERT-specific binding as net mean fluorescence intensity (MFI) which is calculated as the difference between the MFI of Apotel™ binding and binding of the isotype control ± SEM (*n* = 2). *, significantly greater hTERT-specific binding was found after cisplatin treatment in A549 cells when compared with its binding in cisplatin-treated primary bronchial epithelial cells (*P*<0.001, using Tukey's post-test for pairwise comparisons). No significant differences in hTERT-specific binding were found after cell permeablisation.

### DETAILED DESCRIPTION OF THE INVENTION

The present invention is predicated, in part, on the surprising determination that telomerase expression can be detected in dying and dead neoplastic cells and, more particularly, that the induction of neoplastic cell death, for example by a cytotoxic agent such as a DNA damaging reagent, results in an increase in the expression of telomerase. Accordingly, the detection of increased levels of telomerase expression by dead cells, relative to normal levels, provides a convenient and precise mechanism for qualitatively and/or quantitatively assessing dead neoplastic cell levels. These findings therefore provide a highly sensitive and accurate means for assessing a neoplastic condition in a mammal, in particular in the context of monitoring the progression of such a condition or assessing the effectiveness of a therapeutic agent or therapeutic regime. Also facilitated is a highly specific means of targetting therapeutic treatments to sites of neoplastic cells and achieving killing of neoplastic cells via a bystander killing mechanism.

Accordingly, disclosed is a method for detecting non-viable neoplastic cells in a subject, said method comprising screening for the level of telomerase protein and/or gene expression by non-viable cells in said subject or in a biological sample derived from said subject wherein an increase in the level of non-viable cells expressing said telomerase relative to normal levels is indicative of the presence of non-viable neoplastic cells.

Reference to a cell being "non-viable" should be understood as a reference to the subject cell being dead or dying. In relation to the latter, some killing mechanisms result in a series of stages leading to complete cell death. For example, apoptosis is marked by a series of cellular events which occur subsequently to the onset of the apoptotic signal but prior to final cell death. Reference to "dying" is intended to encompass reference to any cell which has received a signal or other stimulus which has resulted in commitment to the cell death events. Without limiting the present invention to any one theory or mode of action, neoplastic cells are generally characterised by both unlimited replicative potential and evasion of apoptosis that would otherwise be induced by DNA damage and failure of checkpoint controls. Nevertheless, even in the absence of a therapeutic treatment regime, a tumour mass will usually be characterised by a percentage of dead or dying cells which may result, for example, from the known heterogeneity of tumour blood supply. More commonly, however, neoplastic cell death is induced via exogenous means such as therapeutic radiotherapy or chemotherapy. The mechanisms by which these treatment regimes achieve neoplastic cell death are variable and depend on the specific nature of the treatment regime which as been selected for use. Although DNA damage resulting from ionising radiation and/or cytotoxic drugs would normally produce apoptosis of the affected cell, an intrinsic feature of malignant cells is the disabling of apoptosis pathways. Therefore, it would be expected that malignant cells would be resistant to the pro-apoptotic effects of DNA damaging agents. Nonetheless, cell death often still occurs in response to treatment with ionising radiation and/or cytotoxic drugs because alternative mechanisms of cell death are activated such as necrosis, mitotic catastrophe, autophagy and premature sensecence. Hence, the induction of neoplastic cell apoptosis is a less common outcome of these widely used treatment regimes than previously believed. Recent data indicate that if the malignant cell does not die an early death from apoptosis then there will be time for DNA repair. However, if there is lack of repair or misrepair of DNA that is sensed during mitosis then post-mitotic cell death will occur (Brown and Attardi, 2004, Nature Reviews Cancer 5:231-237). However, the induction of neoplastic cell apoptosis is a common outcome in the context of any of the more widely used treatment regimes. Reference to an "apoptotic" cell should be understood as a reference to a cell which is undergoing, or has undergone, apoptosis. Without limiting the present invention to any one theory or mode of action, apoptosis is an active process requiring metabolic activity by the dying cell. Apoptosis is often characterised by shrinkage of the cell, cleavage of the DNA into fragments (which give a "laddering pattern" on gels) and by condensation and margination of chromatin.

Unfortunately, due to the relatively non-specific effects of the treatment regimens (in particular systemically administered chemotherapy) rapidly dividing cellular populations (normal and malignant), the patent may experience severe treatment related toxicities. Accordingly, the design of means for both more accurately and rapidly monitoring the effectiveness of a treatment regime (or even monitoring the effectiveness of the endogenous immune response) is of significant value in that it provides a more effective means of assessing and/or tailoring a treatment regime. In the context of the present application, it should therefore be understood that a "non-viable" neoplastic cell is one which has been rendered non-viable by any means, including both apoptotic and non-apoptotic means. An example of apoptotic non-viable neoplastic cells are these which have been killed via administration of a drug which induces cellular apoptosis while an example of non-apoptotic non-viable neoplastic cells are those which have been lysed via the classical complement pathway. The non-viable neoplastic cell detected in the present invention is a dead neoplastic cell.

The present invention therefore provides an *in vitro* method for detecting dead neoplastic cells in a subject undergoing a cell treatment regime, said method comprising screening for the level of telomerase RNA, mRNA or protein in dead cells in a biological sample derived from said subject wherein an increase in the level of telomerase in the dead cells relative to a control level of telomerase is indicative of the presence of dead neoplastic cells.

The present invention more particularly provides an *in vitro* method for detecting dead neoplastic cells in a subject undergoing an anti-neoplastic cell treatment regime, which cell death was induced by an anti-neoplastic cell treatment regime, said method comprising screening for the level of telomerase RNA, mRNA or protein in dead cells in a biological sample derived from said subject wherein an increase in the level of telomerase in the dead cells relative to a control level of telomerase is indicative of the presence of dead neoplastic cells.

Reference to "anti-neoplastic cell treatment regime" should be understood as a reference to any treatment regime which is directed to killing neoplastic cells. The subject treatment regime may be specific or non-specific in this regard. Examples of such treatment regimes include chemotherapy and radiotherapy based regimes.

Reference to a "neoplasm" should be understood as a reference to an encapsulated or unencapsulated growth of neoplastic cells. Reference to a "neoplastic cell" should be understood as a reference to a cell exhibiting abnormal growth. The term "growth" should be understood in its broadest sense and includes reference to proliferation.

The phrase "abnormal growth" in this context is intended as a reference to cell growth which, relative to normal cell growth, exhibits one or more of an increase in the rate of cell division, an increase in the number of cell divisions, a decrease in the length of the period of cell division, an increase in the frequency of periods of cell division or uncontrolled proliferation and evasion of apoptosis. Without limiting the present invention in any way, the common medical meaning of the term "neoplasia" refers to "new cell growth" that results as a loss of responsiveness to normal growth controls, eg. to neoplastic cell growth. Neoplasias include "tumours" which may be either benign, pre-malignant or malignant. The term "neoplasm" should be understood as a reference to a lesion, tumour or other encapsulated or unencapsulated mass or other form of growth which comprises neoplastic cells.

The term "neoplasm", in the context of the present invention should be understood to include reference to all types of cancerous growths or oncogenic processes, metastatic tissues or malignantly transformed cells, tissues or organs irrespective of histopathologic type or state of invasiveness.

The term "carcinoma" is recognised by those skilled in the art and refers to malignancies of epithelial or endocrine tissues including respiratory system carcinomas, gastrointestinal system carcinomas, genitourinary system carcinomas, testicular carcinomas, breast carcinomas, prostate carcinomas, endocrine system carcinomas and melanomas. Exemplary carcinomas include those forming from tissue of the breast. The term also includes carcinosarcomas, e.g. which include malignant tumours composed of carcinomatous and sarcomatous tissues. An "adenocarcinoma" refers to a carcinoma derived from glandular tissue or in which the tumour cells form recognisable glandular structures.

The neoplastic cells comprising the neoplasm may be any cell type, derived from any tissue, such as an epithelial or non-epithelial cell. Reference to the terms "malignant neoplasm" and "cancer" and "carcinoma" herein should be understood as interchangeable.

The term "neoplasm" should be understood as a reference to a lesion, tumour or other encapsulated or unencapsulated mass or other form of growth which comprises neoplastic cells. The neoplastic cells comprising the neoplasm may be any cell type, derived from any tissue, such as an epithelial or non-epithelial cell. Examples of neoplasms and neoplastic cells encompassed by the present invention include, but are not limited to central nervous system tumours, retinoblastoma, neuroblastoma and other paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary, human immunodeficiency associated malignancies (e.g. Kaposi's sarcoma), lymphomas, leukemias, malignant melanomas, sarcomas, endocrine tumours (e.g. of thyroid gland), mesothelioma and other pleural or peritoneal tumours, neuroendocrine tumours and carcinoid tumours.

As detailed hereinbefore, the present invention is predicated on the determination that the induction of neoplastic cell death leads to upregulation of telomerase expression. Without limiting the present invention to any one theory or mode of action, telomerase is a ribonucleoprotein complex which comprises a catalytic subunit protein known as human telomerase reverse transcriptase (hTERT) and 451 base-pair RNA template for telomere extension known as human telomerase integral RNA (hTR). hTERT is the critical or rate limiting enzyme for the activity of telomerase. Telomerase is expressed normally by cells in the body that have high replicative potential such as activated lymphocytes, germ cells that lead to the creation of new organisms and stem cells that are responsible for the ongoing renewal of many tissues even in the adult organism.

Reference to "telomerase" should therefore be understood as a reference to all forms of the telomerase complex or its substituent components hTERT and hTR and to fragments, mutants or variants thereof. It should also be understood to include reference to any isoform which may arise from alternative splicing of hTERT mRNA or mutant or polymorphic forms of hTERT. Reference to "telomerase" is not intended to be limiting and should be read as including reference to all forms of the telomerase complex or its substituent components including any protein, RNA or mRNA form, any subunit polypeptides such as precursor forms which may be generated, whether existing as a monomer, multimer, fusion protein or other complex. Accordingly it should be understood that one may screen for telomerase in its form as a hTERT/hTR complex or as an isolated hTERT or hTR subunit. Preferably, said telomerase is hTERT or hTR.

The present invention therefore preferably provides *an in vitro* method for detecting dead neoplastic cells in a subject undergoing an anti-neoplastic cell treatment regime, said method comprising screening for the level of hTERT protein or mRNA or fragment, mutant or variant thereof in dead cells which have lost membrane integrity in a biological sample derived from said subject wherein an increase in the level hTERT in the dead cells relative to a control level of hTERT is indicative of the presence of dead neoplastic cells.

Preferably, said hTERT is the protein form.

In another preferred embodiment there is provided *an in vitro* method for detecting dead neoplastic cells in a subject undergoing an anti-neoplastic cell treatment regime, said method comprising screening for the level of hTR RNA or fragment, mutant or variant thereof in dead cells which have lost membrane integrity in a biological sample derived from said subject wherein an increase in the level of hTR RNA in the dead cells relative to a control level of hTR RNA is indicative of the presence of dead neoplastic cells.

Preferably, said cell death was induced by an anti-neoplastic cell treatment regime.

More preferably said neoplasm is central nervous system tumours, retinoblastoma, neuroblastoma and other paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary, human immunodeficiency associated malignancies (e.g. Kaposi's sarcoma), lymphomas, leukemias, malignant melanomas, sarcomas, endocrine tumours (e.g. of thyroid gland), mesothelioma and other pleural or peritoneal tumours, neuroendocrine tumours and carcinoid tumours.

Still without limiting the invention in any way, in most eukaryotic cells, the telomere comprises the specialised structure at the linear end of each chromosome, which contains a 5-20 kilobase stretch of the repetitive DNA sequence, TTAGGG, and which is bound by a large number of specific proteins. Although broken chromosomes have sticky ends that fuse to each other, the telomere functions to prevent chromosomal fusions. In recognition of its protective function, the DNA-protein complex at the telomere is also known as the telomeric 'cap'. Conversely, telomere dysfunction or 'uncapping' may manifest as chromosomal end fusions that generate dicentric chromosomes, which may break unevenly during miosis to produce so-called breakage-fusion-bridge cycles. The resulting unequal distribution of genetic material between daughter cells creates genomic instability and may also induce a lethal event known as mitotic catastrophe. Consequently, the specialised DNA-protein complex at the telomere is required to 'cap' or protect the chromosome ends from exposure as double-stranded breaks, which initiates a DNA damage response that may result in DNA repair and, if the damage cannot be repaired, apoptosis of the cell.

The telomere is a complex structure, which incorporates both a novel higher order DNA structure, the T-loop, and a large cast of both telomere associated proteins and telomerase associated proteins. Telomere Repeat binding Factor-2 (TRF2) is a particularly important component of telomere structure and function, which unlike hTERT, is also expressed in all human normal cell types. TRF2 plays an important role in limiting the DNA damage response at telomeres. Other telomere associated proteins, which are expressed in normal cells, include Telomere Repeat binding Factor-1 (TRF1), tankyrase, TRF1 interacting protein (TIN2), hRapl, the Mre11/Rad50/Nbs1 DNA repair complex, Ku70/80 heterodimer. Telomerase associated proteins include the vault protein TEP1, the chaperone proteins p23 and hsp90, the small nucleolar (sno)RNA binding proteins, dyskerin and hGar1, the heterogeneous nuclear ribonucleoproteins (hnRNPs) C1/2, the La ribonucleoprotein and the L22 ribosomal protein and the double stranded RNA binding protein, hStau.

Reference herein to a "subject" should be understood to encompass humans, primates, livestock animals (e.g. sheep, pigs, cattle, horses, donkeys), laboratory rest animals (e.g. mice, rabbits, rats, guinea pigs), companion animals (e.g. dogs, cats) and captive wild animals (e.g. foxes, kangaroos, deer). Preferably, the mammal is a human.

Reference to a "biological sample" should be understood as a reference to any sample of biological material derived from an animal such as, but not limited to, cellular material, biofluids (eg. blood), faeces, tissue biopsy specimens, surgical specimens or fluid which has been introduced into the body of an animal and subsequently removed (such as, for example, the solution retrieved from lung lavage or an enema wash). The biological sample which is tested according to the method of the present invention may be tested directly or may require some form of treatment prior to testing. For example, a biopsy or surgical sample may require homogenisation prior to testing or it may require sectioning for *in situ* testing. Further, to the extent that the biological sample is not in liquid form, (if such form is required for testing) it may require the addition of a reagent, such as a buffer, to mobilise the sample.

To the extent that the target molecule is present in a biological sample, the biological sample may be directly tested or else all or some of the nucleic acid material present in the biological sample may be isolated prior to testing. In yet another example, the sample may be partially purified or otherwise enriched prior to analysis. For example, to the extent that a biological sample comprises a very diverse cell population, it may be desirable to select out a sub-population of particular interest, such as enriching for dead cells or enriching for the cell population of which the neoplastic cell forms part. It is within the scope of the present invention for the target cell population or molecules derived therefrom to be pretreated prior to testing, for example, inactivation of live virus or being run on a gel. It should also be understood that the biological sample may be freshly harvested or it may have been stored (for example by freezing) prior to testing or otherwise treated prior to testing (such as by undergoing culturing).

The choice of what type of sample is most suitable for testing in accordance with the method disclosed herein will be dependent on the nature of the situation, such as the nature of the condition being monitored. Preferably, said sample is of blood, urine, cerebrospinal fluid, pleural or peritoneal effusions and ascites, washings and brushings form oropharynx, lung, biliary tree, colon or bladder, biliary, pancreatic and mammary aspirates, and biopsies and surgical resections.

The present invention is predicated on the unexpected finding that dead neoplastic cells exhibit upregulated levels of telomerase expression. Accordingly, this finding now provides a means of monitoring a neoplastic condition in the context of the levels of dead and dying neoplastic cells which exist at a given point in time. This is particularly useful, for example, in the context of assessing the effectiveness of a therapeutic treatment regime, thereby providing a highly sensitive and rapid means for facilitating the optimisation of a treatment regime. In this regard, the person of skill in the art will understand that one may screen for changes to the levels of telomerase at either the protein (e.g. hTERT) or the encoding nucleic acid molecule level (e.g. hTERT or hTR mRNA). To the extent that it is not always specified, reference herein to screening for the level of "telomerase" should be understood to include reference to screening for either the relevant protein or its encoding primary RNA transcript or mRNA.

As hereinbefore described, telomerase is normally expressed by cells in the body which have high replicative potential such as activated lymphocytes, germ cells and stem cells. Telomerase is not active in most somatic cells. Accordingly, in the context of the present invention, the presence of telomerase expression in the dead cell population of many types of biological samples which are comprised of predominantly somatic cells will, in its own right, be indicative of the presence of dead neoplastic cells. For example, a population of normal peripheral blood mononuclear cells exhibit negligible telomerase levels in the dead cell component. However, in some biological samples, such as lymph node biopsy samples, there may be found higher proportions of non-neoplastic dead cells which are expressing telomerase due to the higher replicative potential required of lymphocytes during an immune response.

Accordingly, it should therefore be understood that the present invention is directed to the correlation of the level of telomerase relative to control levels of this molecule. "Control" levels may be either "normal" levels or the levels obtained from the same patient but at a previous point in time. The "normal" level is the level of telomerase protein or encoding nucleic acid molecule in a biological sample corresponding to the sample being analysed of any individual who has not developed a neoplastic condition. This result therefore provides the background levels, if any, of telomerase which are not due to neoplastic cell death but merely correspond to non-neoplastic dead cells which expressed telomerase due to their high replicative activity. It is expected that in the context of most samples these "normal" or "background" levels will be negligible. The method of the present invention should therefore be understood to encompass all suitable forms of analysis such as the analysis of test results relative to a standard result which reflects individual or collective results obtained from healthy individuals. In a preferred embodiment, said normal reference level is the level determined from one or more subjects of a relevant cohort to that of the subject being screened by the method of the invention. By "relevant cohort" is meant a cohort characterised by one or more features which are also characteristic of the subject who is the subject of screening. These features include, but are not limited to, age, gender, ethnicity, smoker/non-smoker status or other health status parameter. As detailed hereinbefore, said test result may also be analysed relative to a control level which corresponds to an earlier telomerase level result determined from the body fluid of said subject. This is a form of relative analysis (which may nevertheless also be assessed relative to "normal" levels) which provides information in relation to the rate and extent of neoplastic cell death over a period of time, such as during the course of a treatment regime.

Said "normal level" may be a discrete level or a range of levels. Individuals exhibiting dead cell telomerase levels higher than the normal range are generally regarded as having undergone neoplastic cell death, this corresponding to an encouraging prognosis since it may indicate treatment responsiveness and/or the move to a remissive state. In this regard, it should be understood that telomerase levels may be assessed or monitored by either quantitative or qualitative readouts. The reference level may also vary between individual forms of telomerase, such as hTERT vs hTR.

Accordingly, the present invention provides means for assessing both the existence and extent of a population of dead neoplastic cells in a subject. As detailed hereinbefore, this has extremely important implications in terms of assessing the effectiveness of a therapeutic treatment regime. To this end, although a one-off analysis of neoplastic dead cell levels in a biological sample provides information in relation to whether neoplastic cell death has occurred, the present invention is also useful, and particularly valuable, as an ongoing monitor. This can be essential in the context of identifying and monitoring a therapeutic treatment regime where an initial event of neoplastic cell responsiveness to a chemotherapy drug which is being utilised ultimately shifts to neoplastic cell resistance to the chemotherapy drug which is being utilised. The results observed utilising the screening regime herein described may correspond to screening for the existence of a higher level of telomerase over a normal level where a one-off test is being utilised. Alternatively, where a patient is subject to ongoing monitoring and where each successive test result is related to previous results, one may observe a series of increases and decreases in dead cell telomerase expression which map out the on going actions and effectiveness of the treatment regime which has been selected for use. Accordingly, increased dead cell telomerase levels relative to normal levels is indicative of neoplastic cell death - this possibly being due to any one of a number of events including the patient's own immune responsiveness or an effective treatment regime. Increased levels of dead cell telomerase expression relative to a previously analysed sample from that patient may indicate an increasing rate of neoplastic ell death while a decreasing level of telomerase expression in this circumstance may indicate either a loss of effectiveness of the selected treatment regime or the shifting of the patient into a remissive state due to the death and clearance of substantially all neoplastic cells.

Accordingly, disclosed is a method for assessing and/or monitoring a neoplastic condition in a subject, said method comprising screening for the level of telomerase protein and/or gene expression by non-viable cells in said subject or in a biological sample derived from said subject wherein an increase in the level of non-viable cells expressing said telomerase relative to normal levels is indicative of the presence of non-viable neoplastic cells.

There is provided *an in vitro* method for assessing and/or monitoring a neoplastic condition in a subject undergoing an anti-neoplastic cell treatment regime, said method comprising screening for the level of telomerase RNA, mRNA or protein in dead cells which have lost membrane integrity in a biological sample derived from said subject wherein an increase in the level of telomerase in the dead cells relative to a control level of telomerase is indicative of the presence of dead neoplastic cells.

Preferably, said telomerase is hTERT or hTR.

According to this preferred embodiment there is provided *an in vitro* method for assessing and/or monitoring a neoplastic condition in a subject undergoing an anti-neoplastic cell treatment regime, said method comprising screening for the level of hTERT protein or mRNA or fragment, mutant or variant thereof in dead cells which have lost membrane integrity in a biological sample derived from said subject wherein an increase in the level of hTERT in the dead cells relative to a control level of hTERT is indicative of the presence of dead neoplastic cells.

More preferably, said hTERT is the protein form.

In another preferred embodiment there is provided an *in vitro* method for assessing and/or monitoring a neoplastic condition in a subject undergoing an anti-neoplastic cell treatment regime, said method comprising screening for the level of hTR RNA or fragment, mutant or variant thereof in dead cells which have lost membrane integrity in a biological sample derived from said subject wherein an increase in the level of hTR RNA in the dead cells relative to a control level of hTR RNA is indicative of the presence of dead neoplastic cells.

Preferably, said cell death was induced by an anti-neoplastic cell treatment regime.

More preferably, said neoplastic condition is characterised by a neoplasm of the central nervous system tumours, retinoblastoma, neuroblastoma and other paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary, human immunodeficiency associated malignancies (e.g. Kaposi's sarcoma), lymphomas, leukemias, malignant melanomas, sarcomas, endocrine tumours (e.g. of thyroid gland), mesothelioma and other pleural or peritoneal tumours, neuroendocrine tumours and carcinoid tumours.

Disclosed is assessing and/or monitoring the effectiveness of a neoplastic therapeutic treatment regime in a subject said method comprising screening for the level of telomerase protein and/or gene expression by non-viable cells in said subject or in a biological sample derived from said subject wherein an increase in the level of non-viable cells expressing said telomerase relative to normal levels is indicative of the induction of neoplastic cell death.

Preferably, said telomerase is hTERT protein or mRNA or hTR RNA.

In the context and as detailed hereinbefore, an increase in the levels of telomerase expressing dead cells relative to control levels is indicative of the effectiveness of a therapeutic treatment regime which is directed to killing the subject neoplastic cells. Conversely, if telomerase levels remain essentially unchanged relative to normal levels, this would indicate that the subject treatment regime is not being effective. Similarly, a decrease in dead neoplastic cell telomerase levels relative to one or more earlier obtained results may indicate a tapering of or even up-regulation of resistance to the effectiveness of the treatment regime.

The present application should also be understood to disclose a diagnostic method. That is, in addition to using this method to track changes to dead cell populations during and after the course of a therapeutic treatment regime, the existence of a proportion of telomerase expressing dead cells in all tumours even prior to treatment renders this method useful as a diagnostic.

Accordingly, disclosed is a method for diagnosing a neoplastic condition in a subject, said method comprising screening for the level of telomerase protein and/or gene expression by non-viable cells in said subject or in a biological sample derived from said subject wherein an increase in the level of non-viable cells expressing said telomerase relative to normal levels is indicative of the presence of a tumour.

As detailed hereinbefore, one may screen for telomerase at either the protein or mRNA level. To the extent that it is not otherwise specified, reference herein to screening for "telomerase" should be understood to include reference to screening for either the telomerase complex or telomerase subunit protein or its encoding primary RNA transcript or mRNA.

Means of screening for changes in telomerase levels in a subject, or biological sample derived therefrom, can be achieved by any suitable method, which would be well known to the person of skill in the art. Briefly, one may seek to detect hTERT protein or the integral human telomerase RNA (hTR) in a biological sample. Anti-telomerase immuno-interactive molecules may be used to identify hTERT protein directly or provide a means for the isolation of the entire telomerase RNP complex, which may be assayed subsequently for hTERT catalytic activity and/or hTR RNA. Anti-La immuno-interactive molecules provide an indirect means for isolating the entire telomerase RNP complex, which may be assayed subsequently for hTERT catalytic activity and/or hTR RNA. In terms of *in vivo* analyses, anti-telomerase immuno-interactive molecules could be coupled to medical imaging agents in order to visualise specific binding to dead cancer cells, in particular, following the administration of anti-cancer treatments. More specifically, these methods include, but are not limited to:
(i) *In vivo* detection of telomerase. Molecular Imaging may be used following administration of imaging probes or reagents capable of disclosing altered expression levels of the telomerase RNA, mRNA or protein expression product in the biological sample.
   Molecular imaging (Moore et al., BBA, 1402:239-249, 1988; Weissleder et al., Nature Medicine, 6:351-355, 2000) is the *in vivo* imaging of molecular expression that correlates with the macro-features currently visualized using "classical" diagnostic imaging techniques such as X-Ray, computed tomography (CT), MRI, Positron Emission Tomography (PET) or SPECT. In one embodiment, the interactive molecule is coupled to a nuclear medicine imaging agent such as Indium-III or Technetium-99 or to PET imaging agents to MRI imaging agents such as nanoparticles. In another example, one might include the coupling of enzymes as detection agents, for example ADEPT-like where the analyte is generated *in vivo* after binding of the telomerase-interactive molecule to the target and after injection of the enzyme substrate.
(ii) Analysis of RNA expression in the dead cells by Fluorescent *In Situ* Hybridization (FISH), or in extracts from the dead cells by technologies such as Quantitative Reverse Transcriptase Polymerase Chain Reaction (QRTPCR) or Flow cytometric qualification of competitive RT-PCR products (Wedemeyer et al., Clinical Chemistry 48: 9 1398-1405, 2002) or array technologies.

For example, a labelled polynucleotide encoding telomerase may be utilized as a probe in a Northern blot of an RNA extract obtained from a biological sample. Preferably, a nucleic acid extract from the subject is utilized in concert with oligonucleotide primers corresponding to sense and antisense sequences of a polynucleotide encoding telomerase, or flanking sequences thereof, in a nucleic acid amplification reaction such as RT PCR, real time PCR or SAGE. A variety of automated solid-phase detection techniques are also appropriate. For example, very large scale immobilized primer arrays (VLSIPS™) are used for the detection of nucleic acids as, for example, described by Fodor et al., 1991 (Science 251(4995):767-73) and Kazal *et al.,* 1996. The above genetic techniques are well known to persons skilled in the art.

For example, to detect telomerase encoding RNA transcripts, RNA is isolated from a cellular sample suspected of containing neoplastic cells. RNA can be isolated by methods known in the art, e.g. using TRIZOL™ reagent (GIBCO-BRL/Life Technologies, Gaithersburg, Md.). Oligo-dT, or random-sequence oligonucleotides, as well as sequence-specific oligonucleotides can be employed as a primer in a reverse transcriptase reaction to prepare first-strand cDNAs from the isolated RNA. Resultant first-strand cDNAs are then amplified with sequence-specific oligonucleotides in PCR reactions to yield an amplified product.

"Polymerase chain reaction" or "PCR" refers to a procedure or technique in which amounts of a preselected fragment of nucleic acid, RNA and/or DNA, are amplified as described in U.S. Patent No. 4,683,195. Generally, sequence information from the ends of the region of interest or beyond is employed to design oligonucleotide primers. These primers will be identical or similar in sequence to opposite strands of the template to be amplified. PCR can be used to amplify specific RNA sequences and cDNA transcribed from total cellular RNA. See generally Mullis et al., 1987; (Methods Enzymol 155:335-50) and Erlich, 1989 (J Clin Immunol 9(6):437-47). Thus, amplification of specific nucleic acid sequences by PCR relies upon oligonucleotides or "primers" having conserved nucleotide sequences wherein the conserved sequences are deduced from alignments of related gene or protein sequences. For example, one primer is prepared which is predicted to anneal to the antisense strand and another primer prepared which is predicted to anneal to the sense strand of a cDNA molecule which encodes telomerase.

To detect the amplified product, the reaction mixture is typically subjected to agarose gel electrophoresis or other convenient separation technique and the relative presence of telomerase specific amplified nucleic acid detected. For example, the telomerase amplified nucleic acid may be detected using Southern hybridization with a specific oligonucleotide probe or comparing its electrophoretic mobility with nucleic acid standards of known molecular weight. Isolation, purification and characterization of the amplified telomerase nucleic acid may be accomplished by excising or eluting the fragment from the gel (for example, see references Lawn *et al.,* 1981; Goeddel *et al.,* 1980), cloning the amplified product into a cloning site of a suitable vector, such as the pCRII vector (Invitrogen), sequencing the cloned insert and comparing the sequence to the known sequence of telomerase. The relative amounts of telomerase mRNA and cDNA can then be determined.
(iii) Measurement of altered telomerase protein levels in cell extracts or blood or other suitable biological sample, either qualitatively or quantitatively, for example by immunoassay, utilising immunointeractive molecules such as monoclonal antibodies (e.g. anti-telomerase or anti-La where La has interacted with telomerase).

In one example, one may seek to detect telomerase -immunointeractive molecule complex formation. For example, an antibody according to the invention, having a reporter molecule associated therewith, may be utilized in immunoassays. Such immunoassays include but are not limited to radioimmunoassays (RIAs), enzyme-linked immunosorbent assays (ELISAs) and immunochromatographic techniques (ICTs), Western blotting which are well known to those of skill in the art. For example, reference may be made to "Current Protocols in Immunology", 1994 which discloses a variety of immunoassays which may be used in accordance with the present invention. Immunoassays may include competitive assays. It will be understood that the present invention encompasses qualitative and quantitative immunoassays.

Suitable immunoassay techniques are described, for example, in U.S. Patent Nos. 4,016,043, 4,424,279 and 4,018,653. These include both single-site and two-site assays of the non-competitive types, as well as the traditional competitive binding assays. These assays also include direct binding of a labelled antigen-binding molecule to a target antigen. The antigen in this case is telomerase or a fragment thereof

Two-site assays are particularly favoured for use in the present invention. A number of variations of these assays exist, all of which are intended to be encompassed by the present invention. Briefly, in a typical forward assay, an unlabelled antigen-binding molecule such as an unlabelled antibody is immobilized on a solid substrate and the sample to be tested brought into contact with the bound molecule (the antigen is preferably telomerase but may be a telomerase associated protein such as La). After a suitable period of incubation, for a period of time sufficient to allow formation of an antibody-antigen complex, another antigen-binding molecule, suitably a second antibody specific to the antigen, labelled with a reporter molecule capable of producing a detectable signal is then added and incubated, allowing time sufficient for the formation of another complex of antibody-antigen-labelled antibody. Any unreacted material is washed away and the presence of the antigen is determined by observation of a signal produced by the reporter molecule. The results may be either qualitative, by simple observation of the visible signal, or may be quantitated by comparing with a control sample containing known amounts of antigen. Variations on the forward assay include a simultaneous assay, in which both sample and labelled antibody are added simultaneously to the bound antibody. These techniques are well known to those skilled in the art, including minor variations as will be readily apparent.

In the typical forward assay, a first antibody having specificity for the antigen or antigenic parts thereof is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well known in the art and generally consist of cross-linking covalently binding or physically adsorbing, the polymer-antibody complex is washed in preparation for the test sample. An aliquot of the sample to be tested is then added to the solid phase complex and incubated for a period of time sufficient and under suitable conditions to allow binding of any antigen present to the antibody. Following the incubation period, the antigen-antibody complex is washed and dried and incubated with a second antibody specific for a portion of the antigen. The second antibody has generally a reporter molecule associated therewith that is used to indicate the binding of the second antibody to the antigen. The amount of labelled antibody that binds, as determined by the associated reporter molecule, is proportional to the amount of antigen bound to the immobilized first antibody.

An alternative method involves immobilizing the antigen in the biological sample and then exposing the immobilized antigen to specific antibody that may or may not be labelled with a reporter molecule. Depending on the amount of target and the strength of the reporter molecule signal, a bound antigen may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

From the foregoing, it will be appreciated that the reporter molecule associated with the antigen-binding molecule may include the following:-
(a) direct attachment of the reporter molecule to the antibody;
(b) indirect attachment of the reporter molecule to the antibody; i.e., attachment of the reporter molecule to another assay reagent which subsequently binds to the antibody; and
(c) attachment to a subsequent reaction product of the antibody.

The reporter molecule may be selected from a group including a chromogen, a catalyst, an enzyme, a fluorochrome, a chemiluminescent molecule, a paramagnetic ion, a lanthanide ion such as Europium (Eu³⁴), a radioisotope including other nuclear tags and a direct visual label.

In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes suitable for use as reporter molecules is disclosed in U.S. Patent Nos. U.S. 4,366,241, U.S. 4,843,000, and U.S. 4,849,338. Suitable enzymes useful in the present invention include alkaline phosphatase, horseradish peroxidase, luciferase, β-galactosidase, glucose oxidase, lysozyme, malate dehydrogenase and the like. The enzymes may be used alone or in combination with a second enzyme that is in solution.

Suitable fluorochromes include, but are not limited to, fluorescein isothiocyanate (FITC), tetramethylrhodamine isothiocyanate (TRITC), R-Phycoerythrin (RPE), and Texas Red. Other exemplary fluorochromes include those discussed by Dower et al., International Publication No. WO 93/06121. Reference also may be made to the fluorochromes described in U.S. Patent Nos. 5,573,909 (Singer *et a*l), 5,326,692 (Brinkley et al). Alternatively, reference may be made to the fluorochromes described in U.S. Patent Nos. 5,227,487, 5,274,113, 5,405,975, 5,433,896, 5,442,045, 5,451,663, 5,453,517, 5,459,276, 5,516,864, 5,648,270 and 5,723,218.

In the case of an enzyme immunoassay, an enzyme is conjugated to the second antibody, generally by means of glutaraldehyde or periodate. As will be readily recognised, however, a wide variety of different conjugation techniques exist which are readily available to the skilled artisan. The substrates to be used with the specific enzymes are generally chosen for the production of, upon hydrolysis by the corresponding enzyme, a detectable colour change. Examples of suitable enzymes include those described *supra.* It is also possible to employ fluorogenic substrates, which yield a fluorescent product rather than the chromogenic substrates noted above. In all cases, the enzyme-labelled antibody is added to the first antibody-antigen complex, allowed to bind, and then the excess reagent washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample.

Alternately, fluorescent compounds, such as fluorescein, rhodamine and the lanthanide, europium (EU), may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. The fluorescent-labelled antibody is allowed to bind to the first antibody-antigen complex. After washing off the unbound reagent, the remaining tertiary complex is then exposed to light of an appropriate wavelength. The fluorescence observed indicates the presence of the antigen of interest. Immunofluorometric assays (IFMA) are well established in the art and are particularly useful for the present method. However, other reporter molecules, such as radioisotope, chemiluminescent or bioluminescent molecules may also be employed.
(iv) Determining altered protein expression based on any suitable functional test, enzymatic test or immunological test in addition to those detailed in point (iii) above.
(v) Nanotechnology-related techniques such as those outlined in Ferrari (Nature Reviews Cancer 5:161-171, 2005) and Duncan (Nature Reviews Drug Discovery, 2:347-360, 2003)

It should also be understood that the method of the present invention can be performed as an isolated test or it can be combined with any other suitable diagnostic test which may provide additional diagnostic or prognostic information. For example, and without limiting the application of the present invention in any way, the method of the present invention may be performed together with a technology such as CellSearch®, which efficiently and robustly identifies low frequencies of circulating tumour cells in peripheral blood.

Disclosed is a diagnostic kit for a biological sample comprising an agent for detecting telomerase or a nucleic acid molecule encoding telomerase and reagents useful for facilitating the detection by said agent. The agent may be an antibody or other suitable detection molecule.

Disclosed is the use of an interactive molecule directed to telomerase in the manufacture of a quantitative or semi-quantitative diagnostic kit to detect dead neoplastic cells in a biological sample from a patient. The kit may come with instructions for use and may be automated or semi-automated or in a form which is compatible with an automated machine or software.

In addition to the clear diagnostic benefits of the method disclosed herein, the ability to accurately target neoplastic cells now provides a means of delivering therapeutic treatments in a localised and highly targeted manner. To date, such treatments (often referred to as "magic bullets") have not been possible. In particular, in the context of tumour therapy the notion of targeted treatments has not been possible due to the fact that it has not been possible to identify suitable tumour specific antigens against which an antibody could be directed. However, the present disclosure overcomes these shortcomings by directing the therapeutic treatment to the dead cells which comprise the subject tumour. As detailed hereinbefore, even in the absence of any treatment, a tumour will usually comprise a proportion of dead cells which can be utilised to provide the target for the therapeutic treatment. Even micrometastases, which reduce the chances that primary treatment such as mastectomy for breast cancer or colectomy for bowel cancer will be curative, contain apoptotic cells. Micrometastases are deposits of cancer usually less than 1mm in diameter, which are believed to lie dormant because the rate of tumour cell proliferation balances the rate of tumour cell apoptosis. To the extent that a therapeutic regime is underway, the disclosed method provides an opportunity to design and deliver a second phase treatment, for example a more highly toxic treatment, which is able to be more specifically targeted to the regions of dead neoplastic cells which have been induced by the first round of treatment, but which first round treatment may not have been adequate to induce remission. Still further, this method provides a means for administering a less toxic first round treatment in order to upregulate dead neoplastic cell numbers at the sites of tumours, thereby providing a means to accurately target a significantly more toxic second round treatment. This may provide a means of reducing the systemic side effects which are usually associated with chemotherapy. By selecting therapeutic effector mechanisms which can be coupled to an anti-telomerase interactive molecule (such as La or an anti-telomerase antibody), but which function on cells located proximally to the dead cells, that is the viable tumour cells, effective killing of the tumour can be achieved. The subject effector mechanism may take any suitable form but will preferably deliver a toxic molecule or otherwise kill the proximally located viable tumour cells.

Accordingly, disclosed is a method of treating a neoplastic condition in a subject said method comprising administering to said subject an effective amount of an interactive molecule directed to telomerase or antigenic portion thereof, which interactive molecule is linked, bound or otherwise associated with an effector mechanism, for a time and under conditions sufficient to treat said condition.

Preferably, said telomerase is hTERT protein or mRNA or hTR RNA.

More preferably, said interactive molecule is an immunointeractive molecule and even more preferably an anti-telomerase antibody.

Preferably, said neoplastic condition is characterised by nervous system tumours, retinoblastoma, neuroblastoma and other paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary, human immunodeficiency associated malignancies (e.g. Kaposi's sarcoma), lymphomas, leukemias, malignant melanomas, sarcomas, endocrine tumours (e.g. of thyroid gland), mesothelioma and other pleural and peritoneal tumours, neuroendocrine tumours and carcinoid tumours.

Reference to "telomerase", "neoplastic", "non-viable" and "subject" should be understood to have the same meaning as hereinbefore provided.

Disclosed is a method of treating a neoplastic condition in a subject, said method comprising administering to said subject an effective amount of an immunointeractive molecule directed to hTERT protein or antigenic portion thereof, which immunointeractive molecule is linked, bound or otherwise associated with an effector mechanism, for a time and under conditions sufficient to inhibit, reduce or otherwise down-regulate the growth of the neoplasm.

Disclosed is a method of treating a neoplastic condition in a subject, said method comprising administering to said subject an effective amount of an immunointeractive molecule directed to hTERT or hTR RNA or antigenic portion thereof, which immunointeractive molecule is linked, bound or otherwise associated with an effector mechanism, for a time and under conditions sufficient to inhibit, reduce or otherwise down-regulate the growth of the neoplasm.

Preferably said neoplastic condition is a malignant tumour.

Reference to an "effector mechanism" should be understood as a reference to any suitable mechanism which, when localised to the site of dead cells, either directly or indirectly treats the neoplastic condition in issue, for example, down-regulating the growth of adjacent viable tumour cells. In this context, the effector mechanism is most likely a proteinaceous or non-proteinaceous molecule or group of molecules which achieve this outcome. Examples of effector mechanisms suitable for use in the disclosed method include, but are not limited to:
(i) Use of an antibody which has been linked to a cytokine, chemokine or other factor, such as macrophage, dendritic cell and/or T cell activators which act to induce or enhance one or more aspects of an immune response, thereby augmenting bystander killing. For example, the chemotactic peptide, N-formyl-methionyl-leucyl-phenylalanine (FMLP) (Morikawa et al. Cancer Immunol Immunother 27(1):1-6, 1988) and the novel bacterial lipopeptide, JBT 2002 (Shinohara et al. J Immunother 23(3):321-331, 2000) are both activators of tumour associated macrophages.
(ii) Use of an antibody which has been conjugated to a toxin.

Reference to "toxin" should be understood as a reference to any suitable proteinaceous or non-proteinaceous molecule which achieves the object of providing a signal which reduces, prevents or otherwise inhibits the proliferation, differentiation or maintenance of the subject cell (herein referred to as "down-regulating the growth" of said cell). The subject toxin may act by a variety of means including providing its signal via direct contact with a subject cell or emitting a molecule or particle, such as radiation in the case of a radioactive isotope toxin, which provides the signal to the subject cell. Preferably the toxin is a radioisotope and even more preferably a radioisotope which is highly toxic over a short range and exhibits a short half life thereby minimizing the occurrence of inadvertent toxicity on proximally located non-target cells. Most particularly, said radioisotope is an alpha particle emitting radioisotope. However, it should be understood that radioisotopes are not limited to alpha particle emitting radioisotope and may include beta- and gamma-emitting radioisotopes, depending on the clinical context. Examples of alpha-emitting radioisotopes suitable for use in the disclosed method include, but are not limited to, Tb-149, Bi-213 or Thorium-229. It should be understood that the toxin which is utilised in the disclosed method may be in a purified, partially purified or unpurified form. It may also form a component of a larger molecule. The toxin may be naturally occurring or it may be synthetically or recombinantly produced.

Other examples of molecules which should be understood to fall within the scope of "toxin" include ricin, colicheamicin, prodrugs (as antibody-directed prodrug converting enzyme therapy [ADEPT]) and novel biotherapeutic agents, such as catalytic antibodies.

It should be understood that the disclosed method may be performed either *in vivo* or *in vitro.* Examples of *in vitro* applications include, but are not limited to, the *in vitro* purging of biological samples comprising neoplastic cells. For example, bone marrow and/or blood may be removed from a patient, purged to kill tumour cells in accordance with the method of the present invention and then returned to the patient. Such procedures are currently performed, albeit in a significantly less targeted manner, and may reduce the risks of relapse due to transfer of contaminating malignant cells.

Reference to an effector mechanism being "linked, bound or otherwise associated" with an anti-telomerase antibody or other immunointeractive molecule should be understood as a reference to any covalent or non-covalent interactive mechanism which achieves linking of the two molecules. This includes, but is not limited to the use of peptide bonds, ionic bonds, hydrogen bonds, van Der Waals forces or any other interactive bonding mechanism.

Reference to "growth" of a cell or neoplasm should be understood as a reference to the proliferation, differentiation and/or maintenance of viability of the subject cell, while "down-regulating the growth" of a cell or neoplasm is a reference to the process of cellular senescence or to reducing, preventing or inhibiting the proliferation, differentiation and/or maintenance of viability of the subject cell. Preferably the subject growth is proliferation and the subject down-regulation is killing. In this regard, killing may be achieved either by delivering a fatal hit to the cell or by delivering to the cell a signal which induces the cell to apoptose.

Reference herein to "therapeutic" or "prophylactic" treatment is to be considered in its broadest context. The term "treatment" does not necessarily imply that a subject is treated until total recovery. Similarly, "prophylaxis" does not necessarily mean that the subject will not eventually contract a disease condition. Accordingly, treatment and prophylaxis include amelioration of the symptoms of a particular condition or preventing or otherwise reducing the risk of developing a particular condition. The term "prophylaxis" may be considered as reducing the severity or onset of a particular condition. "Treatment" may also reduce the severity of an existing condition.

Without limiting the present disclosure to any one theory or mode of action anti-cancer treatments usually kill by apoptosis but in many cases of advanced cancer, some cancer cells are resistant to the apoptosis that may be induced by a particular anti-cancer treatment. These apoptosis-resistant tumour cells are the source of clinical relapse of disease that ultimately kills most patients with advanced cancers and a significant proportion of those patients with earlier stage cancers. In those patients with advanced cancers who could be shown to be responding to the initial modality of treatment because tumour cell kill could be documented *in vivo,* additional gains in survival and quality of life may be made if another non-cross resistant treatment modality were also to be employed. Therefore, diagnosis of responding cancer patients using the disclosed method can identify those patients who could benefit from supplementary treatment with therapeutic conjugates or hybrid fusion proteins, as detailed.

The use of an anti-telomerase antibody may also be favoured in the adjuvant clinical setting. Although early stage breast and colon cancers, for example, can both be cured by surgery, the risk of overt and incurable systemic relapse is heightened because, in those patients whose primary tumour has certain high-risk features and/or whose regional lymph nodes contain metastases, undetectable systemic micrometastases may already exist. So the use of adjuvant chemotherapy and/or adjuvant hormonal therapy (in the case of breast cancer in particular) cures an additional minor proportion of these patients presumably because the systemic micrometastases are cleared successfully.

Further, although dormant tumours remain small because they lack a blood supply, the tumour cells within the lesion turnover at a rapid rate with the rate of cell division balancing the rate of apoptosis. Therefore, even dormant tumours will be suitable targets for the present technology. Both in the case of clinically evident metastases and micrometastases, apoptosis-resistant cancer cells can be admixed with susceptible cancer cells. Bystander killing of these surviving cancer cells can occur if a non-cross resistant and/or synergistic means of tumour killing were delivered to nearby cancer cells that had been rendered apoptotic by the first treatment. Additional technologies that arm this technology with bystander killing potential can improve its therapeutic efficacy.

A most preferred disclosure is therefore directed to the treatment of a metastatic cancer.

According to this preferred disclosure, there is contemplated a method of therapeutically treating a metastatic cancer in a subject, said method comprising administering to said subject an effective amount of an interactive molecule directed to telomerase or antigenic portion thereof, which interactive molecule is linked, bound or otherwise associated with a therapeutic effector mechanism, for a time and under conditions sufficient to inhibit, reduce or otherwise down-regulate the growth of said metastatic cancer.

As detailed hereinbefore, the present disclosure should also be understood to extend to the down-regulation of growth of neoplastic cells in *an in vitro* environment. For example, neoplastic cells may be purged from an inoculum of bone marrow or peripheral blood stem cells before an autologous transplant.

An "effective amount" means an amount necessary at least partly to attain the desired response, or to delay the onset or inhibit progression or halt altogether, the onset or progression of a particular condition being treated. The amount varies depending upon the health and physical condition of the individual to be treated, the taxonomic group of individual to be treated, the degree of protection desired, the formulation of the composition, the assessment of the medical situation, and other relevant factors. It is expected that the amount will fall in a relatively broad range that can be determined through routine trials.

The present disclosure further contemplates a combination of therapies, such as the administration of the antibody together with subjection of the mammal to circulating cytotoxic agents or to radiotherapy in the treatment of cancer. In another example, the synergistic interactions between a radionuclide and a radiosensitising drug is of particular importance. For instance, a radioimmunotherapy regime can be designed in which there are do-administered an anti-telomerase antibody conjugated to a radionuclide that emits ionising radiation and a radiosensitising drug such as gemcitabine.

Administration of the interactive molecule (herein referred to as the "modulatory agent"), in the form of a pharmaceutical composition, may be performed by any convenient means. The modulatory agent of the pharmaceutical composition is contemplated to exhibit therapeutic activity when administered in an amount which depends on the particular case. The variation depends, for example, on the human or animal and the modulatory agent chosen. A broad range of doses may be applicable. Considering a patient, for example, from about 0.1mg to about 1mg of modulatory agent may be administered per kilogram of body weight per day. Dosage regimes may be adjusted to provide the optimum therapeutic response. For example, several divided doses may be administered continuously, daily, weekly, monthly or other suitable time intervals or the dose may be proportionally reduced as indicated by the exigencies of the situation.

The modulatory agent may be administered in a convenient manner such as by the oral, intravenous (where water soluble), intraperitoneal, intramuscular, subcutaneous, intradermal or suppository routes or implanting (e.g. using slow release molecules). The modulatory agent may be administered in the form of pharmaceutically acceptable nontoxic salts, such as acid addition salts or metal complexes, e.g. with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be administered in tablet form, the tablet may contain a binder such as tragacanth, corn starch or gelatin; a disintegrating agent, such as alginic acid; and a lubricant, such as magnesium stearate.

Routes of administration include, but are not limited to, respiratorally, intratracheally, nasopharyngeally, intravenously, intraperitoneally, subcutaneously, intracranially, intradermally, intramuscularly, intraoccularly, intrathecally, intracereberally, intranasally, infusion, orally, rectally, *via* IV drip patch and implant.

In accordance with these methods, the agent defined in accordance with the present disclosure may be coadministered with one or more other compounds or molecules. By "coadministered" is meant simultaneous administration in the same formulation or in two different formulations via the same or different routes or sequential administration by the same or different routes. For example, the subject agent may be administered together with an agonistic agent in order to enhance its effects. By "sequential" administration is meant a time difference of from seconds, minutes, hours or days between the administration of the two types of molecules. These molecules may be administered in any order.

Disclosed is the use of an anti-telomerase interactive molecule conjugated to an effector mechanism in the manufacture of medicament for the treatment of a neoplastic condition in a subject wherein said effector mechanism treats said condition.

Preferably, said interactive molecule is an immunointeractive molecule and even more preferably an anti-telomerase antibody, such as a monoclonal antibody.

Preferably, said neoplastic condition is characterised by central nervous system tumours, retinoblastoma, neuroblastoma and other paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary, human immunodeficiency associated malignancies (e.g. Kaposi's sarcoma), lymphomas, leukemias, malignant melanomas, sarcomas, endocrine tumours (e.g. of thyroid gland), mesothelioma and other pleural or peritoneal tumours, neuroendocrine tumours and carcinoid tumours.

Disclosed is a pharmaceutical composition comprising the modulatory agent as hereinbefore defined together with one or more pharmaceutically acceptable carriers and/or diluents. Said agents are referred to as the active ingredients.

The pharmaceutical forms suitable for injectable use include sterile aqueous solutions (where water soluble) or dispersions and sterile powders for the extemporaneous preparation of sterile injectable solutions or dispersion or may be in the form of a cream or other form suitable for topical application. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilisation. Generally, dispersions are prepared by incorporating the various sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.
When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Disclosed compositions or preparations are prepared so that an oral dosage unit form contains between about 0.1µg and 2000mg of active compound.

The tablets, troches, pills, capsules and the like may also contain the components as listed hereafter: a binder such as gum, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

Yet another disclosure relates to the agent as hereinbefore defined, when used in the method of the present invention.

The present invention is further defined by the following non-limiting examples:

### EXAMPLE 1

### Materials and Methods

### In vitro culture and apoptosis induction of human Peripheral blood mononuclear cells and human Jurkat T cell leukemia cells.

Peripheral blood mononuclear cells (PBMC) were prepared from a buffy coat of a discarded unit of whole blood that had been obtained from the South Australian Red Cross Blood Bank. Initially, PBMC had been separated by density gradient centrifugation over Lymphoprep (Axis-Shield, Dundee, UK) and frozen in aliquots using a controlled rate freezer. Subsequently, the aliquoted cells were thawed and again purified over Lymphoprep to obtain viable cells. PBMC were grown in RPMI supplemented with 10% foetal calf serum (FCS), 50U/mL penicillin and 50µg/mL streptomycin sulphate (JRH Biosciences, Lenexa, KA, USA) for 72h. Half of the culture was treated with the T cell mitogen, conconavalin A (ConA) (Sigma, St Louis, MO, USA), 10µg/mL, and half of the culture was left untreated. After culture, cells were again purified over Lymphoprep to remove dead cells and rendered apoptotic by a further 24h culture with 1µM staurosporine (STS) (Sigma). Jurkat cells were rendered apoptotic by 24h culture in RPMI supplemented with 10% FCS, 50U/mL penicillin and 50µg/mL streptomycin sulphate and 0.5µM STS. For studies of primary monocytic and lymphocytic cells derived from PBMC, PBMC were first purified using the Lymphoprep^{®} separation protocol. Then PBMC were cultured in RPMI-1640 with 5% FCS overnight to separate adherent (predominantly monocytic cells) from suspension cells (predominantly T lymphocytes). Suspension cells were collected and incubated in RPMI-1640 with 5% FCS in the presence or absence of 1µM STS and Jurkat cells were similarly incubated in the presence or absence of 0.5µM STS. Adherent monocytic cells and U937 cells (a monocytic lymphoma cell line) were incubated in RPMI-1640 with 5% FCS in the presence or absence of 1µM and 0.5µM of STS, respectively. After 24h and 48h, untreated (control) and STS-treated cells were collected and the control cells were permeabilised using 2% paraformaldehyde solution followed by a 1:10 dilution with methanol at -20°C for 5 min.

After treatment, all cells were harvested, washed in PBS and stained with 7-amino actinomycin D (7-AAD) (2µg/mL) and anti-La 3B9 mAb or its isotype control mAb, Sal5, and then with the secondary anti-mouse antibody conjugated to R-phycoerythrin (Zymed, San Francisco, CA, USA), or with rat anti-hTERT mAb (Alexis Biochemicals, San Diego, CA, USA) and then with the secondary anti-rat antibody conjugated to FITC (Chemicon). Cells were analysed using a FACScan (Becton Dickinson, Franklin Lakes, NJ, USA) and the results analysed with WinMDI v2.8 software.

### Cytotoxic treatment of malignant human cell lines and subsequent detection of hTERT in apoptotic malignant and primary cells.

The Jurkat and Raji cell lines were cultured in RPMI supplemented with 10%FCS, 50U/mL penicillin and 50µg/mL streptomycin sulphate and the U2OS and HeLa cell lines were cultured in DMEM supplemented with 10%FCS, 50U/mL penicillin and 50µg/mL streptomycin sulphate (JRH Laboratories). The cell lines were treated for 24 or 48-hour periods with various cytotoxic agents as follows: Jurkat and Raji cells with etoposide, 20µg/mL; HeLa and U2OS cells with cisplatin, 1µg/mL, and vincristine, 0.1µg/mL; Jurkat, HeLa and U2OS cells with 0.5µM STS, and Raji cells with 2µM STS. The treated cells were stained with propidium iodide (PI) (Sigma) 0.5µg/mL and FITC-conjugated Sal5 or 3B9 mAb, or mouse anti-TRF2 mAb (clone 36, Becton-Dickinson) and then secondary antibody, anti mouse FITC (Chemicon, Temecula, CA, USA), or rat anti-hTERT mAb (clone 36-10, Alexis Biochemicals) and then secondary antibody, anti rat FITC (Caltag, Burlingame, CA, USA). Purified mouse IgG or rat IgG (Institute of Medical and Veterinary Science, Adelaide, Australia) were used as negative controls for TRF2 and hTERT staining, respectively. To further improve the method for detection of hTERT for later use in whole blood staining, which appeared to have been hindered by the presence of autofluorescence presumably emanating from red blood cells, anti-hTERT monoclonal antibody clone 36-10 was conjugated directly to AlexaFluor350 (Molecular Probes), which is excited at 355nm and emits maximally between 420-460nm. Anti-hTERT-Alexa350 was combined with the EpCAM monoclonal antibody (BD Biosciences), which is conjugated to PE that excites at 488nm and emits 560-590nm, and the nuclear impermeant DNA-binding dye TOPRO-3 (Molecular Probes), which excites at 560nm and emits maximally in the far red region (> 650 nm). This combination of fluorochromes did not require compensation of signals as each of these molecules is excited using a different laser and the emissions are collected using separate filters on the high throughput LSR-II flow cytometer (BD Biosciences).

### Results

Anti-La (monoclonal antibody hybridoma 3B9) binding was compared to apoptotic PBMC, which are mainly T cells, and apoptotic Jurkat cells, which is a human T cell leukemia line. It is clear that the fluorescence intensity is greater for apoptotic Jurkat cells than PBMC (Figure 3), which suggests that La is more highly expressed in dead and dying tumour cells.

The human T cell leukemia cell line, Jurkat, and the human B lymphoma cell line, Raji, were treated for 24 or 48 hour periods with the following cytotoxic agents: staurosporine, which is a pan-kinase inhibitor, etoposide, which is a topoisomerase II inhibitor, vincristine, which is a microtubule depolymerising agent and cisplatin, which is a DNA cross linking agent. Cells were then stained with propidium iodide (PI) and the anti-La monoclonal antibody (mAb), 3B9, or its isotype control, Sal5, or with mAbs specific for hTERT or TRF2. As negative controls for staining with the hTERT- or TRF2-specific mAbs, rat IgG and anti-rat secondary antibody or mouse IgG and anti-mouse secondary antibody, respectively, were used.

Using flow cytometry, it was found that the various cytotoxic agents induced cell death in both Jurkat (Figure 4A) and Raji cells (Figure 4C) as soon as 24 hours after cytotoxic treatment as evidenced by binding of PI and 3B9. Similarly, TRF2 staining of PI⁺ dead Jurkat cells was evident as soon as 24 hours after cytotoxic treatment (Figure 4D) and 48 hours afterwards (Figure 4E). Only slightly increased staining for TRF2 was observed for both vincristine and cisplatin-treated Raji cells at 48 hours (arrows, Figure 4F). In contrast, it took 48 hours after treatment with cisplatin, etoposide and vincristine for hTERT staining of PI⁺ dead Jurkat cells to be evident (arrows, Figure 4B). hTERT staining of PI⁺ dead Raji cells was evident 48 hours after treatment with vincristine (arrow, Figure 4C). As additional controls, the telomerase-expressing human cervical carcinoma cell line, HeLa, and the telomerase-negative human osteosarcoma cell line, U2OS (M Frolkis et al. Cancer Gene Therapy 10, 239-249, 2003), were also treated with staurosporine, vincristine and etoposide and analysed by flow cytometry 48 hours after treatment. Evident staining for hTERT and TRF2 was observed in PI⁺ dead HeLa cells (Figure 4G) but not in PI⁺ dead U2OS a cells (Figure 4H). The increased expression of telomerase protein after cytotoxic drug-induced apoptosis of cancer cells may represent a feedback response to the telomere uncapping, which has been induced by DNA-damaging drugs.

Human lymphocytes, in particular activated lymphocytes, also express telomerase. As illustrated in Figure 5, little if any telomerase expression was observed in dead PBMC, which are predominantly lymphocytes, or in Jurkat cells 24 hours after induction of apoptosis using staurosporine. Figure 6 illustrates that permeabilisation of Jurkat cells either as the result of fixation artefact or cytotoxic insult was required to detect binding of 36-10 mAb to hTERT protein. Interestingly, fixation of etoposide-treated Jurkat cells revealed additional sites for 36-10 mAb binding which were not evident in unfixed and treated cells until 48 hours after treatment. Moreover, fixation of Jurkat cells that had not been treated with etoposide (control) appeared to result in still greater exposure of the determinant recognised by 36-10 mAb. Together with the lack of binding of 36-10 mAb to PI-intermediate Jurkat cells, which have features of apoptotic bodies (Figure 4B), these data indicate that the conformation of hTERT and/or its association with other proteins alters during programmed cell death to restrict the availability of the 36-10 mAb binding site (Figure 6). Permeabilisation of malignant or primary cells demonstrates that the malignant Jurkat and U937 cells demonstrated significantly higher levels of hTERT expression than their corresponding primary cell counterparts, lymphocytic and monocytic cells, respectively (Figure 7). Treatment of these cell types with staurosporine to induce apoptosis and post-apoptotic cell permeabilisation shows that this malignant cell still exhibits elevated levels of hTERT expression, which indicates that hTERT represents a suitable tumour-associated antigen for preferential tumour targeting.

The cytotoxic drug, etoposide, was also used to treat H69, which is a cell line derived from the chemoresponsive tumour known as small cell lung cancer (SCLC), to demonstrate that the extent of cell death is dose-dependent (Figure 8). The monoclonal antibody BerEP4 recognises a determinant on the epithelial cell adhesion molecule (EpCAM), which is borne by many types of epithelial cells and their malignant counterparts. Propidium Iodide (PI) is a nuclear impermeant nucleic acid-binding fluorochrome so that staining of cells with PI signifies loss of cell membrane integrity as a result of cell death. Hence, BerEP4⁺ PI⁺ can be positively identified as dead H69 carcinoma cells, the maximal proportion of which was achieved at an etoposide concentration of 200-400µg/mL (Figure 8A). Subsequently, it was demonstrated that the optimal concentration of anti-hTERT monoclonal antibody, clone 36-10, required to detect upregulation of hTERT protein expression in the dead H69 cells after cytotoxic treatment was 50µg/mL (Figure 9B). In contrast, the equivalent concentration of isotype control monoclonal antibody did not produce significant staining of dead H69 cells after cytotoxic treatment (Figure 9A). H69 cells, which had been killed with etoposide and which were identified as BerEP4⁺ 7-AAD⁺, were detected at a dilution of 1:50 among normal peripheral blood mononuclear cells (PBMC) (Figure 10). However, compensation steps were needed to ensure detection of etoposide-treated malignant cells among the background of normal PBMC. This method of detection of etoposide-treated malignant cells was improved upon by using a combination of fluorochromes, which would not require compensation in the flow cytometer, to label DNA, hTERT and EpCAM and thereby more robustly identify etoposide-treated malignant cells (Figure 11).

### EXAMPLE 2

### Material and Methods

### Cytotoxic drug treatment of cancer cell lines in vitro

H69 cells were cultured in RPMI-1640 containing 5% fetal calf serum (FCS) and 400µg/mL etoposide. Aliquots were removed at 24h, 48h and 72h, and washed with phosphate-buffered saline (PBS) and then stained for cytofluographic analysis.

### Enrichment of circulating tumour cells from blood of a small cell lung cancer (SCLC) patient

Blood samples (2.5mL) collected in heparinised tubes were enriched for expression of the BerEP4 epithelial cell adhesion molecule (EpCAM) using the CELLection™ Epithelial Enrich kit as per the manufacturer's instructions (Dynal^{®} Biotech, Invitrogen Corp., USA). Briefly, 250µL BerEP4-beads were mixed with the blood sample for 30 min. at RT. Beads were bound to a Dynal^{®} magnet and washed 5 times with PBS. Bead-bound cells were released from the magnet using PBS containing 0.1% bovine serum albumin (BSA) and deoxyribonuclease I (DNase I) as described by the manufacturer. The released cells were removed to a fresh tube, centrifuged, and washed with PBS. Samples were incubated for 30 min. at RT with anti-mouse IgG (2µg/mL) to block any mouse BerEP4 antibody remaining on the isolated cells then washed with PBS before being stained and analysed by flow cytometry.

### Preparation of whole blood samples for cytofluographic analysis

Red cells in the heparinised peripheral blood samples (2.5mL) were lysed after diluting the blood 1:10 in red blood cell lysis buffer (8 g/L ammonium chloride and 1.2 g/L Tris-HCl, pH 7.2) and constantly rotating the diluted samples for 15 min at RT. Then cells were pelleted at 3,000 xg for 10 min. and washed with PBS before being stained and analysed by flow cytometry.

### Cytofluographic analysis

Cells were incubated for 30 min. at room temperature (RT) with monoclonal antibodies (mAb) at a concentration of 50µg/mL: APOTEL™, which is a rat anti-hTERT mAb (clone 36-10; Alexis Biochemicals), or a matching isotype (IgG₂ₐκ) control mAb of irrelevant specificity. Cells were washed with PBS and incubated for 30 min. at RT with a biotin-conjugated (Fab')₂ fragment of an anti-rat IgG antibody at a concentration of 2µg/mL. Cells were washed and incubated for 15 min. at RT with streptavidin-PE or with streptavidin-FITC at a concentration of 2µg/mL. Finally, cells were washed with PBS and analysed by flow cytometry (FACScan, Becton-Dickinson) after incubation for 15 min with 2µg/mL 7-amino-actinomycin D (7-AAD) at RT.

### Statistical analysis

Statistical analyses were performed using GraphPad Prism v.4.0 software.

### Results

The ability of a monoclonal antibody (mAb), which specifically recognises the human telomerase reverse transcriptase (hTERT), was studied in the context of its ability to bind dead cells of small cell lung cancer (SCLC) both *in vitro* and *in vivo* after cell death is induced using cytotoxic drugs. First, the SCLC cell line, H69, which expresses the BerEP4 marker of Epithelial Cell Adhesion Molecule (EpCAM) was studied. H69 was cultured *in vitro* and treated with etoposide to induce cell death. Loss of viability of the cancer cells was evident 24h after cytotoxic drug treatment because they bound the DNA-binding dye, 7-AAD (Figure 12A). Although binding of APOTEL™, which is an hTERT specific monoclonal antibody (mAb), to the dead H69 cells was observed at 24, 48 and 72 hours after cytotoxic drug treatment, specific APOTEL™ binding was significantly increased 48 hours after treatment (Figure 1B).

Next, the binding of APOTEL™ in the peripheral blood of a 73-year-old male patient, GP, who had extensive-stage SCLC was studied. GP, who had not previously been treated for his cancer, was administered cytotoxic chemotherapy using carboplatin at AUC5 on day 1 together with etoposide 120mg/m² on days 1, 2 and 3. Heparinised peripheral blood samples were drawn from the patient before chemotherapy (0h) and 24, 48 and 72 hours after initiation of chemotherapy. To demonstrate that GP's blood contained circulating tumour cells (CTC), his blood was enriched before and after cytotoxic drug treatment for BerEP4-expressing cells, which were not present in the peripheral blood of a normal healthy volunteer (Figure 13, upper row of panels). Forty-eight hours after cytotoxic chemotherapy, there was a large increase in the number of dead (7-AAD⁺) CTC, which clearly and specifically bound APOTEL™ (Figure 13, middle and lower row of panels). In addition, whole blood samples were obtained from GP before and after cytotoxic chemotherapy and were not subject to further manipulation except red cell lysis before analysis for viability using 7-AAD and for expression of hTERT among 7-AAD⁺ cells. The greatest increase of 7-AAD⁺ cells in GP's blood was observed 48 hours after initiation of cytotoxic chemotherapy and the 7-AAD⁺ cells had the highest binding of APOTEL™ (Figure 14). Together, as was demonstrated by *in vitro* culture of SCLC cells, these results indicate that not only does cytotoxic chemotherapy induce the death of circulating SCLC cells but it also augments expression of hTERT in the dead tumour cells, 48 hours after the cytotoxic stimulus (Figure 15 and 16).

Although the laboratory findings have not yet been correlated with a formal radiological evaluation of overall tumour response to chemotherapy, GP did have a palpable and painful sternal mass before cytotoxic chemotherapy, which became markedly less painful and shrunk significantly in size within two weeks of cytotoxic chemotherapy administration.

These results indicate that APOTEL™ identifies cancer cells that have died as the result of cytotoxic drug administration and, hence, APOTEL™ is useful for predicting early chemotherapy responses in cancer patients.

### Discussion

In addition to its use in isolation, the APOTEL™ test may also be usefully integrated with the only FDA-approved technology for the detection of circulating tumour cells (CTC), which is an immunomagnetic bead-based method of enumerating circulating tumour cells (CTC) called CellSearch® (Veridex, Warren, NJ, USA). The system is designed to count epithelial cells, which are first separated from the blood by BerEP4-specific antibody-coated magnetic beads. CTC are identified among the isolated cells after fluorescence labelling with the double stranded DNA-specific dye, 4,6-diamidino-2-phenylindole (DAPI), and mAb specific for leukocytes (CD45-allophycocyanin) and epithelial cells (cytokeratin 8-, 18- and 19-phycoerythrin) and analysis using the CellSpotter Analyzer® (Veridex). The technology facilitates specific detection of CTC as shown by a large survey of normal individuals and of individuals with malignant and non-malignant diseases. One of 344 (0.3%) individuals, who were healthy or who had non-malignant diseases, had ≥2 CTC per 7.5 mL of blood. In 2,183 blood samples from 964 metastatic carcinoma patients, CTC ranged from 0 to 23,618 CTC per 7.5 mL (mean, 60 ± 693 CTC per 7.5 mL), and 36% (781 of 2,183) of the specimens had ≥2 CTC. Detection of ≥2 CTC occurred at the following rates: 57% (107 of 188) of prostate cancers, 37% (489 of 1,316) of breast cancers, 37% (20 of 53) of ovarian cancers, 30% (99 of 333) of colorectal cancers, 20% (34 of 168) of lung cancers, and 26% (32 of 125) of other cancers (Allard et al. Clin Cancer Res 10:6897-6904, 2004).

After the initial step of BerEP4 enrichment, the CellSearch® technology enables the detection of cells as shown in a study of the blood of prostate cancer patients. Here, few CTC were intact and most appeared as damaged cells or cell fragments by microscopy. By flow cytometry, these CTC events stained variably with DAPI and frequently expressed the apoptosis-induced, caspase-cleaved cytokeratin-18 (Larson et al. Cytometry Part A 62A, 46-53, 2004).

In a study of CTC in patients with metastatic breast cancer, none of 145 normal control subjects had >2 CTC per 7.5mL of blood whereas in 177 cancer patients >2 CTC per 7.5mL of blood were found in 61% of patients. Nonetheless, a cut-off of 5 CTC per 7.5mL of blood was chosen to distinguish patients with an unfavourable prognosis from patients with a favourable prognosis. The results of this study indicated that the number of CTC before treatment was an independent predictor of progression-free survival and overall survival in patients with metastatic breast cancer (Cristofanilli et al. New Engl J Med 351:781-791,2004).

A further report of this study was published that analysed the significance of CTC found in patients with metastatic breast cancer receiving first-line chemotherapy. In these patients, the presence of ≥5 CTC in 7.5mL of peripheral blood before the initiation of first-line chemotherapy was associated with short progression free survival (PFS) and overall survival (OS). Median OS at this time was >18 months for patients with <5 CTC *vs.* 14.2 months for patients with ≥5 CTC. Moreover, the greatest statistically different differences in median OS were observed for the subset of patients (22%) with visceral disease who were receiving chemotherapy and who were negative for hormone receptors and HER2/*neu.* At the first follow-up blood draw approximately one month post-treatment, the median PFS for patients with <5 CTC was 9.5 months *vs.* 2.1 months for patients with ≥5 CTC (*P*=0.0057), and the median OS for patients with <5 CTC was more than 18 months *vs.* 11.1 months for patients with ≥5 CTC (*P*=0.0012). At the first follow-up imaging visit at approximately 9 weeks post-treatment, the median PFS for patients with <5 CTC was 8.9 months *vs.* 1.8 months for patients with ≥5 CTC (*P*=0.0001), and the median OS for patients with <5 CTC was 18 months *v* 11.1 months for patients with ≥5 CTC (*P*=0.0001). These data indicated that detection of CTC 3-4 weeks post-treatment predicted treatment efficacy, which was determined by medical imaging studies 8-10 weeks post-treatment (Cristofanilli et al. J Clin Oncol. 23:1420-1430, 2005).

However, because APOTEL™ detects dead CTC 48 hours after initiation of cytotoxic chemotherapy, it can give a much more prompt indication of an effective and survival-prolonging chemotherapy response than other methods such as the number of CTC 3-4 weeks post-treatment or the results of medical imaging studies 8-10 weeks post-treatment.

### EXAMPLE 3

### Materials and Methods

### Materials

Cell culture media, RPMI 1640, DMEM and Trypsin EDTA were obtained from JRH Biosciences Inc (Lexona KS, USA). Staurosporine and ethidium bromide were obtained from Sigma-Aldrich Co. (St Louis, MO, USA) Trizol and Superscript II were purchased from Invitrogen (Carlsbad, CA, USA) and Amplitaq Gold from Applied Biosystems (Foster City, CA, USA) All primers were manufactured by Geneworks (Adelaide, SA)

### Cell lines and culture

Jurkat cells (ATCC #TIB-152) were maintained in RPMI 1640 supplemented with 5% fetal calf serum (FCS). U2OS cells (ATCC #HTB-96) were maintained in DMEM supplemented with 5% FCS and passaged every 48-72 hours after detachment with Trypsin EDTA. Jurkat cells express telomerase and thus have the integral RNA component, hTR, whereas U2OS cells lack telomerase and hTR. Apoptosis was induced by the addition of 0.5µM staurosporine to the culture media for 24,48 or 72 hours.

### RNA extraction and reverse transcription

Cells were harvested and washed in PBS before RNA extraction of 5x10⁶ cells with Trizol according to manufacturer's instructions. RNA was resuspended in 10ul of DEPC water and the concentration determined. Three micrograms of RNA was reverse transcribed using Superscript II and oligo dT according to the manufacturer's instructions.

### hTR PCR

PCR was performed with primers specific for hTR. Two sets of primers were used to generate PCR products of 111bp (Chen XQ et al. Clin Cancer Res 6:3823-3826, 2000) and 153 bp (Bodnar AG et al. Exp Cell Res 228:58-64, 1996) in size:
hTR fwd 5' -GAAGGGCGTAGGCGCCGTGCTTTTGC (SEQ ID NO:1)
hTR rev 5' -GTTTGCTCTAGAATGAACGGTGGAAGG (Chen *et al., supra*) (SEQ ID NO:2)
hTR fwd 5' -GCCTGGGAGGGGTGGTGGCTATTTTTTG (Bodnar *et al., supra*) (SEQ ID NO:3) together with Chen reverse primer.

A control set of PCR primers was used to amplify mRNA of the housekeeping gene, GAPDH:
GAPDH fwd 5' -CGGAGTCAACGGATTTGGTCGTAT (SEQ ID NO:4)
GAPDH rev 5' -AGCCTTCTCCATGGTGGTGAAGAC (308bp PCR product) (SEQ ID NO:5)

One microlitre of cDNA was subjected to the polymerase chain reaction (PCR) in a 25µL reaction containing 1xPCR buffer, 10nmol MgCl₂ and 5nmol dNTPs, 50pmol of each primer and 0.625U of Amplitaq gold. The reaction conditions for PCR amplification were as follows: an initial denaturation of 95°C for 7 minutes, followed by 30 cycles of 95°C for 30 seconds, 55°C for 30 seconds and 72°C for 1 minute, with a final extension of 72°C for 5 minutes. PCR products were electrophoresed on 1% agarose and visualised by ethidium bromide staining. The negative control for the PCR was water together with primers.

### Results

RT-PCR amplification of GAPDH shows detectable mRNA in both cells lines up to 72 hours after the induction of apoptosis. Viability analysis using trypan blue staining indicated that of U2OS cells, 36%, 95% and 100% were dead at 24, 48 and 72 hours, respectively, and of Jurkat cells, 40%, 85% and 100% were dead at 24, 48 and 72 hours, respectively. The RNA for hTR was not detected in the hTR-negative U2OS cells but was detected in the hTR-positive Jurkat cells 24, 48 and 72 hours after the induction of apoptosis.

### Discussion

These data indicate that it is possible to PCR-amplify RNA including that of GAPDH and hTR from dead cells *in vitro*. There is evidence to indicate that RNA including mRNA is sequestered inside new intracellular fibro-granular structures called Heterogenous Ectopic RNP-Derived Structures (HERDS) that develop during apoptosis. HERDS are extruded into the cytoplasm and move toward the cell surface as part of apoptotic blebs, which are eventually released as apoptotic bodies. Although the formation of HERDS was shown to be a reversible marker of transcriptional arrest, which may occur during processes such as heat shock and other physical stress, nutrient deprivation and use of DNA damaging drugs, HERDS formation and the commitment to apoptosis becomes irreversible when nucleolar proteins are identified in HERDS (Biggiogera et al. Biol Cell 96:603-615, 2004). To date there have been no reports of labelling of the La antigen in HERDS. However, since La can bind hTR, immunoaffinity isolation of La from dead cells should facilitate the PCR amplification of hTR particularly when tumour cell numbers are limiting such as in biofluids, which may include tumour cells circulating in the peripheral blood of even early-stage cancer patients. QZyme™ technology (Clontech) is an example of a quantitative PCR-based assay system, which could usefully be applied to La-bound hTR isolated by immunoaffinity methods, because it is unaffected by biosample complexity and requires no optimisation.

### Materials and Methods

### Cell permeabilisation

Clonetics^{®} conditioned primary cells and normal human bronchial epithelium with retinoic acid (NHBE) were obtained from Cambrex Corporation (NJ, USA) and maintained in culture using the commercially supplied medium. Cancer cell lines were obtained from ATCC and cultured in recommended media. At confluence, cells were detached using Trypsin-EDTA solution, washed with PBS and permeablised by incubating 5x10⁶ cells/mL with 2% paraformaldehyde for 10min. at RT then diluting 1:10 with -20°C absolute methanol.

### Cytotoxic treatment

Cell death was induced by adding cisplatin to the culture medium at a concentration of 20µg/mL for 48 hours.

### Flow cytometry

Cells were collected, washed with PBS and incubated for 30min. at RT with APOTEL™ or matched isotype control mAb of irrelevant specificity at 50µg/mL. Cells were washed with PBS and incubated for 30min. at RT with anti-rat IgG Alexa₄₈₈ conjugated antibodies at 2µg/mL. Cells were washed and incubated for 10min. at RT with 2µg/mL 7-AAD then analysed by flow cytometry using a B-D FACScan instrument.

### Results

Telomerase expression in primary and malignant bronchial epithelial cells was similar (Fig. 18A&B). After cisplatin treatment, significantly more hTERT was detected by fluorocytometric analysis of hTERT-specific mAb binding of the malignant bronchial epithelial cell line, A549 (Fig. 18C). The apparent increase in hTERT expression in cisplatin-treated bronchial carcinoma cells may result from increased protein synthesis and/or protein release from another subcellular compartment during the intracellular rearrangements characteristic of apoptosis and/or revelation of the anti-hTERT mAb epitope that favours higher binding of the hTERT-specific mAb. This result is relevant clinically because it would predict the behaviour of hTERT-specific mAb binding in humans *in vivo.* Thus, primary cells that are damaged and leaky because of cytotoxic drug treatment will not bind significant amounts of anti-telomerase antibody unlike damaged and leaky carcinoma cells, which are the true targets of anti-cancer treatments.

These *in vitro* results indicate the potential for targeting a hTERT-specific mAb carrying a payload such as a radionuclide to dead cancer cells *in vivo* for imaging and therapeutic purposes.

The invention also includes any and all combinations of any two or more of the features referred to or indicated in this specification.

### BIBLIOGRAPHY:

Allard WJ et al. Clin Cancer Res 10:6897-6904,2004
Biggiogera M et al. Biol Cell 96:603-615, 2004
Bodnar AG et al. Exp Cell Res 228:58-64, 1996
Brown, J.M. and Attardi, L.D. Nature Reviews Cancer 5:231-237, 2005
Chen XQ et al. Clin Cancer Res 6:3823-3826, 2000
Cristofanilli M et al. New Engl JMed 351, 781-791, 2004
Cristofanilli M et al. J Clin Oncol. 23:1420-1430, 2005
Duncan, R. The dawning era of polymer therapeutics. Nature Reviews Drug Discovery. 2:347-360,2003
Erlich HA (1989) J Clin Immunol 9(6):437-47
Ferrari, M. Cancer nanotechnology: opportunities and challenges. Nature Reviews Cancer 5:161-171,2005.
Fodor SP, Read JL, Pirrung MC, Stryer L, Lu AT, Solas D. (1991) Science 251(4995):767-73
Frolkis M. et al. Cancer Gene Therapy 10, 239-249, 2003
Goeddel *et al.,* 1980
Larson CJ et al. Cytometry Part A 62A, 46-53, 2004
Lawn *et al.,* 1981
Moore, A., Basilion, J., Chiocca, E., and Weissleder, R., BBA, 1402:239-249,1988
Morikawa et al. Cancers Immunol Immunother 27(1):1-6, 1988
Mullis KB, Faloona FA. (1987) Methods Enzymol 155:335-50
Shinohara et al. J Immunother 23(3):321-331, 2000
Wedemeyer, N., Potter, T., Wetzlich, S. and Gohde, W. Clinical Chemistry 48:9 1398-1405,2002
Weissleder, R., Moore, A., Ph.D., Mahmood-Bhorade, U., Benveniste, H., Chiocca, E.A., Basilion, J.P. Nature Medicine, 6:351-355, 2000

## Claims

1. An *in vitro* method for detecting dead neoplastic cells in a subject undergoing an anti-neoplastic cell treatment regime, said method comprising screening for the level of telomerase RNA, mRNA or protein in dead cells which have lost membrane integrity in a biological sample derived from said subject wherein an increase in the level of telomerase in the dead cells relative to a control level of telomerase is indicative of the presence of dead neoplastic cells.

2. An *in vitro* method for assessing or monitoring a neoplastic condition in a subject undergoing an anti-neoplastic cell treatment regime, said method comprising screening for the level of telomerase RNA, mRNA or protein in dead cells which have lost membrane integrity in a biological sample derived from said subject wherein an increase in the level of telomerase in the dead cells relative to a control level of telomerase is indicative of the presence of dead neoplastic cells.

3. An anti-telomerase immuno-interactive molecule or imaging probe capable of disclosing altered levels of the telomerase RNA, mRNA or protein expression product for use in an *in vivo* method of detecting dead neoplastic cells in a subject undergoing an anti-neoplastic cell treatment regime, said method comprising using said molecule or probe to screen for the level of telomerase expression in dead cells which have lost membrane integrity in said subject, wherein an increase in the level of telomerase in the dead cells relative to a control level of telomerase is indicative of the presence of dead neoplastic cells.

4. An anti-telomerase immuno-interactive molecule or imaging probe capable of disclosing altered levels of the telomerase RNA, mRNA or protein expression product for use in an *in vivo* method of assessing or monitoring a neoplastic condition in a subject undergoing an anti-neoplastic cell treatment regime, said method comprising using said molecule or probe to screen for the level of telomerase expression in dead cells which have lost membrane integrity in said subject, wherein an increase in the level of telomerase in the dead cells relative to a control level of telomerase is indicative of the presence of dead neoplastic cells.

5. The method according to or defined in claim 2 or the anti-telomerase immuno-interactive molecule or imaging probe for use according to or defined in claim 4, wherein said method is performed in order to assess or monitor the effectiveness of a neoplasm therapeutic treatment regime in a subject.

6. The method or the anti-telomerase immuno-interactive molecule or imaging probe for use according to or defined in according to or defined in any one of claims 1 to 5 wherein said cell death is induced by an anti-neoplastic cell treatment regime.

7. The method or the anti-telomerase immuno-interactive molecule or imaging probe for use according to or defined in according to or defined in any one of claims 1 to 6 wherein the telomerase molecule which is the subject of detection is selected from:
(i) the telomerase complex;
(ii) hTERT; or
(iii) hTR
or fragment, mutant or variant thereof.

8. The method or the anti-telomerase immuno-interactive molecule or imaging probe for use according to or defined in according to or defined in claim 7 wherein said telomerase molecule is selected from:
(i) hTERT protein;
(ii) hTERT mRNA; or
(iii) hTR RNA
or fragment, mutant or variant thereof.

9. The method or the anti-telomerase immuno-interactive molecule or imaging probe for use according to or defined in according to or defined in any one of claims 3 to 8, wherein the anti-telomerase immuno-interactive molecule is a monoclonal antibody.

10. The method orthe anti-telomerase immuno-interactive molecule or imaging probe for use according to or defined in according to or defined in claim 8 or claim 9, wherein said telomerase molecule is hTERT protein which is detected using an anti-hTERT monoclonal antibody.

11. The method or the anti-telomerase immuno-interactive molecule or imaging probe for use according to or defined in according to or defined in any one of claims 1 to 10 wherein said neoplastic cells are derived from a central nervous system tumour, retinoblastoma, neuroblastoma, paediatric tumour, head or neck cancer such as squamous cell cancer, breast or prostate cancer, lung cancer, kidney cancer, such as renal cell adenocarcinoma, oesophagogastric cancer, hepatocellular carcinoma, pancreaticobiliary neoplasia, such as adenocarcinoma or islet cell tumour, colorectal cancer, cervical cancer, anal cancer, uterine or other reproductive tract cancer, urinary tract cancer, such as of the ureter or bladder, germ cell tumour such as a testicular germ cell tumour or ovarian germ cell tumour, ovarian cancer, such as an ovarian epithelial cancer, carcinoma of unknown primary, human immunodeficiency associated malignancy, such as Kaposi's sarcoma, lymphoma, leukemia, malignant melanoma, sarcoma, endocrine tumour, such as of the thyroid gland, mesothelioma or other pleural or peritoneal tumour, neuroendocrine tumour or carcinoid tumour.

12. The method according to any one of claims 1, 2 or 5 to 11 wherein said biological sample is a sample of blood, urine, cerebrospinal fluid, pleural or peritoneal effusions or ascites, washings or brushings from oropharynx, lung, biliary tree, colon or bladder, biliary, pancreatic or mammary aspirates, or biopsies or surgical resections.

13. The method according to or defined in any one of claims 1 to 12, wherein said control level of telomerase is:
i) the level of telomerase obtained from one or more individuals who have not developed a neoplastic condition; or
ii) the level of telomerase obtained at a previous point in time from said subject.

## Patentansprüche

1. In-vitro-Verfahren zum Erfassen toter neoplastischer Zellen in einem Subjekt, das sich einem Behandlungsschema gegen neoplastische Zellen unterzieht, wobei das Verfahren ein Durchmustern auf den Spiegel von Telomerase-RNA, -mRNA oder -Protein in toten Zellen, die Membranintegrität verloren haben, in einer biologischen Probe umfasst, die von dem Subjekt abgeleitet ist, wobei ein Anstieg im Telomerasespiegel in den toten Zellen relativ zu einem Kontrollspiegel von Telomerase auf das Vorhandensein toter neoplastischer Zellen hinweist.

2. In-vitro-Verfahren zum Bewerten und/oder Überwachen eines neoplastischen Zustands in einem Subjekt, das sich einem Behandlungsschema gegen neoplastische Zellen unterzieht, wobei das Verfahren ein Durchmustern auf den Spiegel von Telomerase-RNA, -mRNA oder -Protein in toten Zellen, die Membranintegrität verloren haben, in einer biologischen Probe umfasst, die von dem Subjekt abgeleitet ist, wobei ein Anstieg im Telomerasespiegel in den toten Zellen relativ zu einem Kontrollspiegel von Telomerase auf das Vorhandensein toter neoplastischer Zellen hinweist.

3. Immun-interaktives Anti-Telomerasemolekül oder Bildgebungssonde, imstande, geänderte Spiegel des Telomerase-RNA-, -mRNA oder -Proteinexpressionsprodukts zu offenbaren, zur Verwendung in einem In-vitro-Verfahren zum Erfassen toter neoplastischer Zellen in einem Subjekt, das sich einem Behandlungsschema gegen neoplastische Zellen unterzieht, wobei das Verfahren die Verwendung des Moleküls oder der Sonde zum Durchmustern auf den Spiegel der Telomeraseexpression in toten Zellen, die Membranintegrität verloren haben, in dem Subjekt umfasst, wobei ein Anstieg im Telomerasespiegel in den toten Zellen relativ zu einem Kontrollspiegel von Telomerase auf das Vorhandensein toter neoplastischer Zellen hinweist.

4. Immun-interaktives Anti-Telomerasemolekül oder Bildgebungssonde, imstande, geänderte Spiegel des Telomerase-RNA-, -mRNA oder -Proteinexpressionsprodukts zu offenbaren, zur Verwendung in einem In-vitro-Verfahren zum Bewerten und/oder Überwachen eines neoplastischen Zustands in einem Subjekt, das sich einem Behandlungsschema gegen neoplastische Zellen unterzieht, wobei das Verfahren die Verwendung des Moleküls oder der Sonde zum Durchmustern auf den Spiegel der Telomeraseexpression in toten Zellen, die Membranintegrität verloren haben, in dem Subjekt umfasst, wobei ein Anstieg im Telomerasespiegel in den toten Zellen relativ zu einem Kontrollspiegel von Telomerase auf das Vorhandensein toter neoplastischer Zellen hinweist.

5. Verfahren nach oder definiert in Anspruch 2 oder immun-interaktives Anti-Telomerasemolekül oder Bildgebungssonde zur Verwendung nach oder definiert in Anspruch 4, wobei das Verfahren zum Bewerten oder Überwachen der Wirksamkeit eines therapeutischen Neoplasma-Behandlungsschemas in einem Subjekt durchgeführt wird.

6. Verfahren oder immun-interaktives Anti-Telomerasemolekül oder Bildgebungssonde zur Verwendung nach oder definiert in einem der Ansprüche 1 bis 5, wobei der Zelltod durch ein antineoplastisches Zellbehandlungsschema herbeigeführt wird.

7. Verfahren oder immun-interaktives Anti-Telomerasemolekül oder Bildgebungssonde zur Verwendung nach oder definiert in einem der Ansprüche 1 bis 6, wobei das Telomerasemolekül, das Gegenstand der Erfassung ist, ausgewählt ist aus:
(i) dem Telomerasekomplex;
(ii) hTERT; oder
(iii) hTR
oder einem Fragment, einer Mutante oder Variante davon.

8. Verfahren oder immun-interaktives Anti-Telomerasemolekül oder Bildgebungssonde zur Verwendung nach oder definiert in Anspruch 7, wobei das Telomerasemolekül ausgewählt ist aus:
(i) hTERT Protein;
(ii) hTERT mRNA; oder
(iii) hTR RNA
oder einem Fragment, einer Mutante oder Variante davon.

9. Verfahren oder immun-interaktives Anti-Telomerasemolekül oder Bildgebungssonde zur Verwendung nach oder definiert in einem der Ansprüche 3 bis 8, wobei das immun-interaktive Anti-Telomerasemolekül ein monoklonaler Antikörper ist.

10. Verfahren oder immun-interaktives Anti-Telomerasemolekül oder Bildgebungssonde zur Verwendung nach oder definiert in Anspruch 8 oder Anspruch 9, wobei das Telomerasemolekül hTERT Protein ist, das mit Hilfe eines monoklonalen Anti-hTERT-Antikörpers erfasst wird.

11. Verfahren oder immun-interaktives Anti-Telomerasemolekül oder Bildgebungssonde zur Verwendung nach oder definiert in einem der Ansprüche 1 bis 10, wobei die neoplastischen Zellen von einem Zentralnervensystemtumor, Retinoblastom, Neuroblastom, pädiatrischen Tumor, Kopf- oder Halskrebs, wie Plattenepithelkarzinom, Brust- oder Prostatakrebs, Lungenkrebs, Nierenkrebs, wie Nierenzellenadenokarzinom, Speiseröhren-Magenkrebs, Leberzellkarzinom, Speiseröhren-Gallengangneoplasien, wie Adenokarzinom oder Inselzelltumor, Dickdarmkrebs, Gebärmutterhalskrebs, Analkrebs, Uteruskrebs oder Krebs anderer Fortpflanzungsorgane, Harnwegekrebs, wie des Harnleiters oder der Blase, Keimzellentumor wie Hodenkeimzellentumor oder Eierstockkeimzellentumor, Eierstockkrebs, wie Eierstockepithelkrebs, Karzinom eines unbekannten primären, humanen Immunmangels in Verbindung mit Malignität, wie Kaposi Sarkom, Lymphom, Leukämie, malignen Melanom, Sarkom, endokrinen Tumor, wie der Schilddrüse, Mesotheliom oder anderen pleuralen oder peritonealen Tumor, Neuroendokrintumor oder karzinoiden Tumor abgeleitet sind.

12. Verfahren nach einem der Ansprüche 1, 2 oder 5 bis 11, wobei die biologische Probe eine Probe von Blut, Harn, Rückenmarksflüssigkeit, pleuralen oder peritonealen Effusionen und Aszites, Waschlösungen und Abstrichen von Mundrachenraum, Lunge, Gallenwegsystem, Dickdarm oder Blase, Galle, Bauchspeicheldrüse- und Brustabsaugungen und chirurgischen Resektionen ist.

13. Verfahren nach oder definiert in einem der Ansprüche 1 bis 12, wobei der Kontrollspiegel von Telomerase:
i) der Telomerasespiegel ist, der von einem oder mehreren Individuen erhalten wird, die keinen neoplastischen Zustand entwickelt haben; oder
ii) der Telomerasespiegel ist, der zu einem früheren Zeitpunkt von dem Subjekt erhalten wurde.

## Revendications

1. Procédé *in vitro* pour détecter des cellules néoplasiques mortes chez un sujet subissant un régime de traitement des cellules antinéoplasiques, ledit procédé comprenant le criblage du niveau d'ARN, d'ARNm ou de protéine de la télomérase dans des cellules mortes qui ont perdu l'intégrité de leur membrane dans un échantillon biologique tiré dudit sujet, dans lequel une augmentation du niveau de la télomérase dans les cellules mortes par rapport à un niveau témoin de la télomérase est indicative de la présence de cellules néoplasiques mortes.

2. Procédé *in vitro* permettant d'évaluer ou de contrôler un état néoplasique chez un sujet subissant un régime de traitement de cellules antinéoplasiques, ledit procédé comprenant le criblage du niveau d'ARN, d'ARNm ou de protéine de la télomérase dans des cellules mortes qui ont perdu l'intégrité de leur membrane dans un échantillon biologique tiré dudit sujet, dans lequel une augmentation du niveau de la télomérase dans les cellules mortes par rapport à un niveau témoin de la télomérase est indicative de la présence de cellules néoplasiques mortes.

3. Molécule immunointeractive ou sonde d'imagerie anti-télomérase capable de décrire des niveaux altérés de l'ARN, de l'ARNm ou d'un produit d'expression protéique de la télomérase pour utilisation dans un procédé *in vivo* de détection de cellules néoplasiques mortes chez un sujet subissant un régime de traitement de cellules antinéoplasiques, ledit procédé comprenant l'utilisation de ladite molécule ou de ladite sonde pour cribler le niveau d'expression de la télomérase dans les cellules mortes qui ont perdu l'intégrité de leur membrane dans ledit sujet, dans laquelle une augmentation du niveau de la télomérase dans les cellules mortes par rapport à un niveau témoin de télomérase est indicative de la présence de cellules néoplasiques mortes.

4. Molécule immunointeractive ou sonde d'imagerie anti-télomérase capable de décrire des niveaux altérés d'ARN, d'ARNm ou d'un produit d'expression de protéine de la télomérase pour utilisation dans un procédé *in vivo* d'évaluation ou de surveillance d'un état néoplasique chez un sujet subissant un régime de traitement de cellules antinéoplasiques, ledit procédé comprenant l'utilisation de ladite molécule ou de ladite sonde pour cribler le niveau d'expression de la télomérase dans les cellules mortes qui ont perdu l'intégrité de leur membrane chez ledit sujet, dans laquelle une augmentation du niveau de la télomérase dans les cellules mortes par rapport à niveau témoin de télomérase est indicative de la présence de cellules néoplasiques mortes.

5. Procédé selon ou comme défini dans la revendication 2 ou molécule immunointeractive ou sonde d'imagerie anti-télomérase pour utilisation selon ou comme défini dans la revendication 4, dans lequel ou laquelle ledit procédé est effectué afin d'évaluer ou de contrôler l'efficacité d'un régime de traitement thérapeutique de néoplasme chez un sujet.

6. Procédé ou molécule immunoréactive ou sonde d'imagerie anti-télomérase pour utilisation selon ou comme défini dans l'une quelconque des revendications 1 à 5, dans lequel ou laquelle ladite mort cellulaire est induite par un régime de traitement de cellules antinéoplasiques.

7. Procédé ou molécule immunoréactive ou sonde d'imagerie anti-télomérase pour utilisation selon ou comme défini dans l'une quelconque des revendications 1 à 6, dans lequel ou laquelle la molécule de télomérase qui fait l'objet de la détection est choisie parmi les suivantes :
(i) le complexe de télomérase ;
(ii) hTERT ; ou
(iii) hTR,
ou un fragment, un mutant ou un variant de celles-ci.

8. Procédé ou molécule immunointeractive ou sonde d'imagerie anti-télomérase pour utilisation selon ou comme défini dans la revendication 7, dans lequel ou laquelle ladite molécule de télomérase est choisie parmi les suivantes :
(i) protéine hTERT ;
(ii) ARNm de hTERT ; ou
(iii) RN de hTR,
ou un fragment, un mutant ou un variant de celles-ci.

9. Procédé ou molécule immunointeractive ou sonde d'imagerie anti-télomérase pour utilisation selon ou comme défini dans l'une quelconque des revendications 3 à 8, dans lequel ou laquelle la molécule immunointeractive anti-télomérase est un anticorps monoclonal.

10. Procédé ou molécule immunointeractive ou sonde d'imagerie anti-télomérase pour utilisation selon ou comme défini dans la revendication 8 ou la revendication 9, dans lequel ou laquelle ladite molécule de télomérase est une protéine hTERT qui est détectée en utilisant un anticorps monoclonal anti-hTERT.

11. Procédé ou molécule immunointeractive ou sonde d'imagerie anti-télomérase pour utilisation selon ou comme défini dans l'une quelconque des revendications 1 à 10, dans lequel lesdites cellules néoplasiques sont tirées d'une tumeur du système nerveux central, d'un rétinoblastome, d'un neuroblastome, d'une tumeur pédiatrique, d'un cancer de la tête ou du cou, tel que l'épithéliome malpighien, le cancer du sein ou de la prostate, le cancer des poumons, le cancer des reins, notamment un adénocarcinome des cellules rénales, un cancer gastro-oesophagien, un carcinome hépatocellulaire, une néoplasie pancréatico-biliaire, telle qu'un adénocarcinome ou un nésidioblastome, un cancer colorectal, un cancer cervical, un cancer anal, un cancer des voies utérines et d'autres voies reproductrices, un cancer des voies urinaires, tel que celui de l'urètre ou de la vessie, les tumeurs embryonnaires et germinales, tels qu'une tumeur des cellules germinales testiculaires ou une tumeur des cellules germinales ovariennes, un cancer des ovaires, tel qu'un cancer épithélial ovarien, un carcinome de malignité associée à une immunodéficience humaine primaire inconnue, notamment le sarcome de Kaposi, un lymphome, une leucémie, un mélanome malin, un sarcome, une tumeur de l'endocrine, notamment de la glande thyroïde, des mésothéliomes ou d'autre tumeurs pleurales ou péritonéales, une tumeur neuro-endocrinale ou une tumeur carcinoïde.

12. Procédé selon l'une quelconque des revendications 1, 2 ou 5 à 11, dans lequel ledit échantillon biologique est un échantillon de sang, d'urine, de fluide cérébrospinal, d'épanchements ou d'ascites pleuraux ou péritonéaux, de lavages ou de brossages venant de l'oropharynx, des poumons, du tronc biliaire, du côlon ou de la vessie, des échantillons aspirés biliaires, pancréatiques ou mammaires ou encore des biopsies ou des résections chirurgicales.

13. Procédé selon ou comme défini dans l'une quelconque des revendications 1 à 12, dans lequel ledit niveau témoin de télomérase est :
i) le niveau de télomérase obtenu à partir d'un ou plusieurs individus qui n'ont pas développé d'état néoplasique ; ou
ii) le niveau de télomérase obtenu dans une période antérieure chez ledit sujet.
